# EUROPEAN PATENT SPECIFICATION

(11) **EP 1 845 787 B1**
(45) Date of publication and mention of the grant of the patent: **15.04.2015**
(21) Application number: 06719601.4
(22) Date of filing: 27.01.2006
(51) Int. Cl.: A61K 47/34, A61K 47/40, A61K 31/05

(54) **FORMULATIONS FOR INJECTION OF CATECHOLIC BUTANES, INCLUDING NDGA COMPOUNDS, INTO ANIMALS**
FORMULIERUNGEN FÜR DIE INJEKTION VON CATHECHOLBUTANEN, DARUNTER NDGA-VERBINDUNGEN, BEI TIEREN
FORMULATIONS POUR L'INJECTION DE BUTANES CATECHOLIQUES, DONT DES COMPOSES NDGA, A DES ANIMAUX

(30) Priority: 27.01.2005 US 647495 P; 27.01.2005 US 647648 P
(43) Date of publication of application: 24.10.2007
(73) Proprietor: Erimos Pharmaceuticals LLC, Houston, TX 77036 (US)
(72) Inventor: LOPEZ, Rocio, Alejandra, Durham, North Carolina 27703 (US); BLOMBERG, Jessica, Andrea, LoDuca, Raleigh, North Carolina 27615 (US); RHODES, Melissa, Claire, Raleigh, North Carolina 27617 (US); HELLER, Jonathan, Daniel, San Francisco, CA 94114 (US)
(74) Representative: Grünecker Patent- und Rechtsanwälte PartG mbB
(86) International application number: PCT/US2006/002807
(87) International publication number: WO 2006/081364

(56) References cited:
- WO-A1-88/01509
- WO-A1-98/15184
- WO-A1-98/15266
- WO-A1-99/17761
- WO-A2-2004/112695
- US-A- 4 774 229
- US-A- 4 880 637
- US-A- 5 541 232
- US-B1- 6 291 524
- US-B2- 6 372 755
- 'Stedman's Medical Dictionary 27th Ed.' INJECTION, [Online] XP008136354 Retrieved from the Internet: <URL:http://www.thomsonhc.com>
- 'Stedman's Medical Dictionary 27th Ed.' PARENTERAL, [Online] XP008109702 Retrieved from the Internet: <URL:http://www.thomsonhc.com>

## Description

### BACKGROUND OF THE INVENTION

This application relates to compositions, including injectible formulations and methods for administration of tetra-O-methyl NDGA, to animals, such as humans, for treatment of diseases, for example, cancer, psoriasis or other proliferative or inflammatory diseases, metabolic diseases such as diabetes or neuronal diseases including neurodegenerative diseases such as Alzheimer's disease, stroke, amyotrophic lateral sclerosis and Parkinson's disease.

Nordihydroguaiaretic acid ("NDGA") has been tested for therapeutic applications in certain experimental animals. For example, Jordan et al. in US 5,008,294 described the effect of NDGA on human mammary carcinoma, MX-1, which was implanted subcutaneously into mice on day 0 (Example 2). Mice that developed tumors were injected with various doses of NDGA on day 1, in a single intratumor injection. The solubilizing solvent for the NDGA in this example was not disclosed. In another example, a stock solution of 10⁻²M NDGA in 4 mL of DMSO (i.e., dimethyl sulfoxide) and 6 mL distilled water was used for *in vitro* testing on cells (Example 5).

Huang et al. tested the therapeutic application of certain derivatives of NDGA (i.e., "NDGA derivatives"), as described in US 6,214,874. In one example, an NDGA derivative was dissolved in DMSO (Example 5).

The use of DMSO for administration into humans has been controversial. Further, use of DMSO has been associated with undesirable side effects such as sedation, headache, nausea, dizziness, burning or aching eyes and noticeable breath odor. (See, for example, Brobyn, R.D., "The human toxicology of dimethyl sulfoxide," Ann. N.Y. Acad. Sci. 243: 497-506, January 27, 1975.) If the catecholic butanes, including NDGA or NDGA derivatives (collectively, "NDGA compounds") are to be useful as therapeutics for humans and other animals, for example, as described in PCT/US2004/016117, published December 29,2004 as International Publication No. WO 04/112696, it would be highly desirable to develop new formulations, other than formulations containing DMSO, for solubilizing such catecholic butanes, including the NDGA compounds such as NDGA derivatives, for example, M₄N.

WO 99/17761 A1 discloses formulations for injection comprising nordihydroguaiaretic acid (NDGA) and an amphiphilic vehicle, in which the amphiphilic vehicle is a mixture of saturated polyglycolyzed glycerides.

WO 2004/112695 A2 discloses compositions for intravenous administration and treatment of obesity comprising catecholic butane with carrier selected from oils such as corn oil.

WO 88/01509 A1 a solution of NDGA in polyethylene glycol 400 which was injected intraperitoneally into nude mice.

Furthermore, it would be desirable if such formulations are not only safe but also stable, have minimal side effects upon administration to animals. It would further be desirable to develop formulations for these compounds that would allow distribution of an effective amount of these compounds to the desired target tissues *in vivo* in humans and other animals. The present invention provides these desirable benefits.

### BRIEF SUMMARY OF THE INVENTION

It is, therefore, one of the objects of the present invention to provide one or more novel formulations for solubilization of the tetra-O-methyl NDGA herein, where such formulations do not contain DMSO and are suitable for injection into animals.

It is another one of the objects to provide formulations as above that are safe and have few adverse side effects when administered to animals, including humans.

It is another one of the objects to provide formulations as one or more of the foregoing that have a commercially reasonable period of stability.

It is a further one of the objects to provide formulations as one or more of the foregoing that can be administered parenterally to animals.

It is another one of the objects to provide for formulations as one or more of the foregoing that have a commercially reasonable half-life in circulation upon administration into animals.

In accordance to the foregoing one or more objects of the invention, there is provided embodiments of the present invention as follows:

A composition for injection into animals comprising an active pharmaceutical ingredient and a pharmaceutically acceptable carrier, wherein the active pharmaceutical ingredient comprises a tetra-O-methyl NDGA, and the carrier comprises propylene glycol, the propylene glycol is in the absence of ascorbic acid or butylated hydroxytoluene ("BHT"), and when the polyethylene glycol is polyethylene glycol 400, the polyethylene glycol 400 is present in the absence of polyethylene glycol 8000 and a cyclodextrin.

The present invention also includes a method of treatment of a disease in a subject comprising: (a) providing the composition of the present invention; and (b) administering the composition by injecting the composition into the subject, wherein the composition comprises an effective amount of the active pharmaceutical ingredient.

Additionally, the present invention includes a kit comprising the composition of the present invention and instructions for use thereof.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the embodiments shown.

In the drawings:

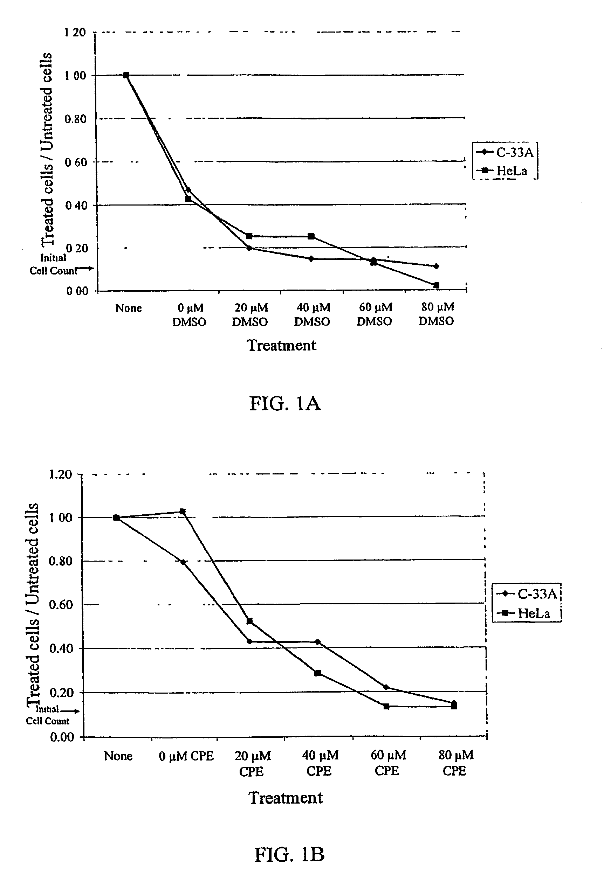

FIG. 1 comprises FIGS. 1A and 1B and depicts the results of cell proliferation assays conducted for the C-33A cell line and the HeLa cell line after M₄N treatment. FIG. 1A is a graphical representation of the ratio of number of cells present after M₄N treatment over the number of cells present in the absence of M₄N treatment, where M₄N was provided in amounts varying from 0 µM to 80 µM in a DMSO formulation. FIG. 1B is a graphical representation of the ratio of number of cells present after M₄N treatment over the number of cells present in the absence of M₄N treatment, where M₄N was provided in amounts varying from 0 µM to 80 µM in a HP-β-CD/PEG formulation ("CPE" formulation).

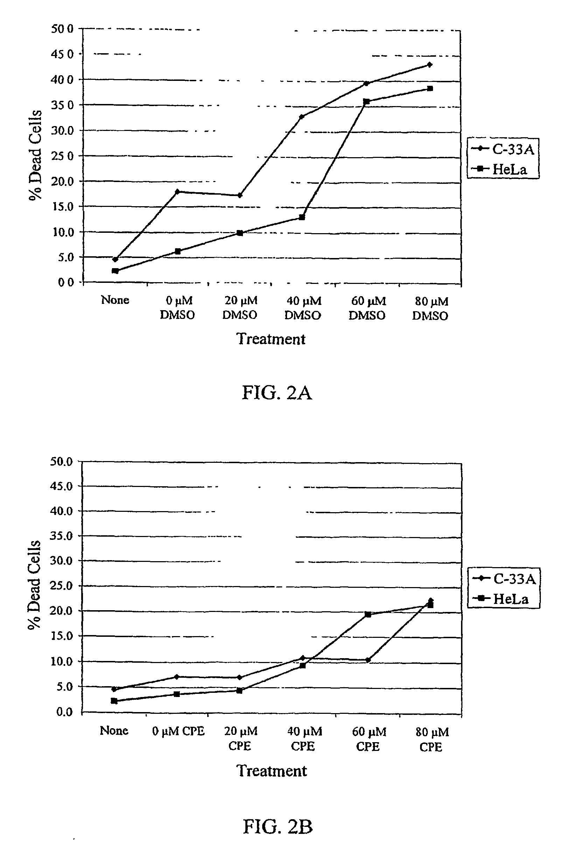

FIG. 2 comprises FIGS. 2A and 2B and is a graphical representation of cell death measurements based on percentage of dead cells for C-33A cells and HeLa cells in the absence or presence of varying concentrations of M₄N in a DMSO (FIG. 2A) formulation or in a HP-β-CD/PEG formulation (FIG. 2B). The M₄N concentrations varied from 0 µM to 80 µM.

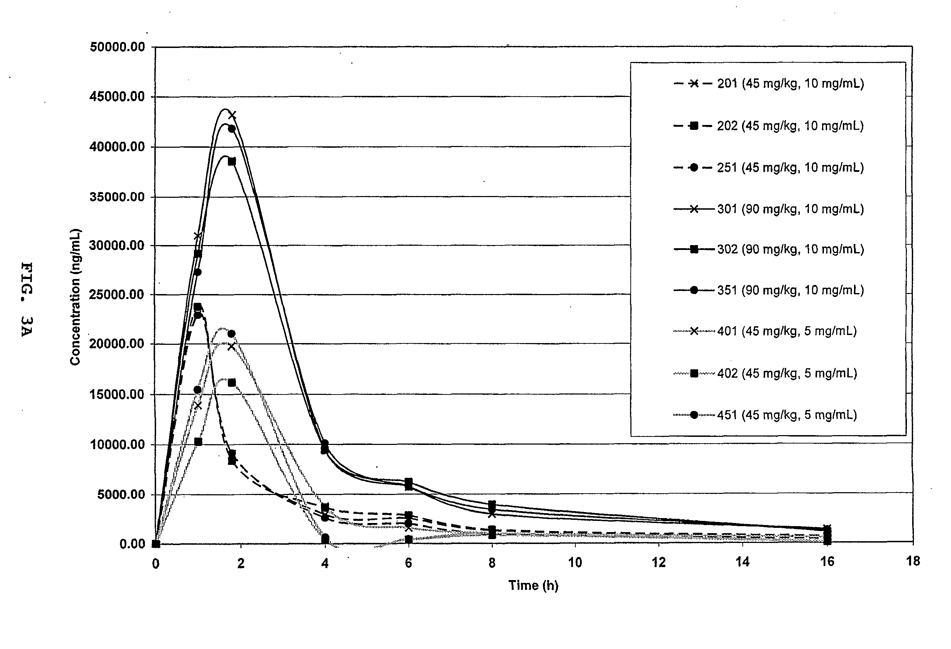

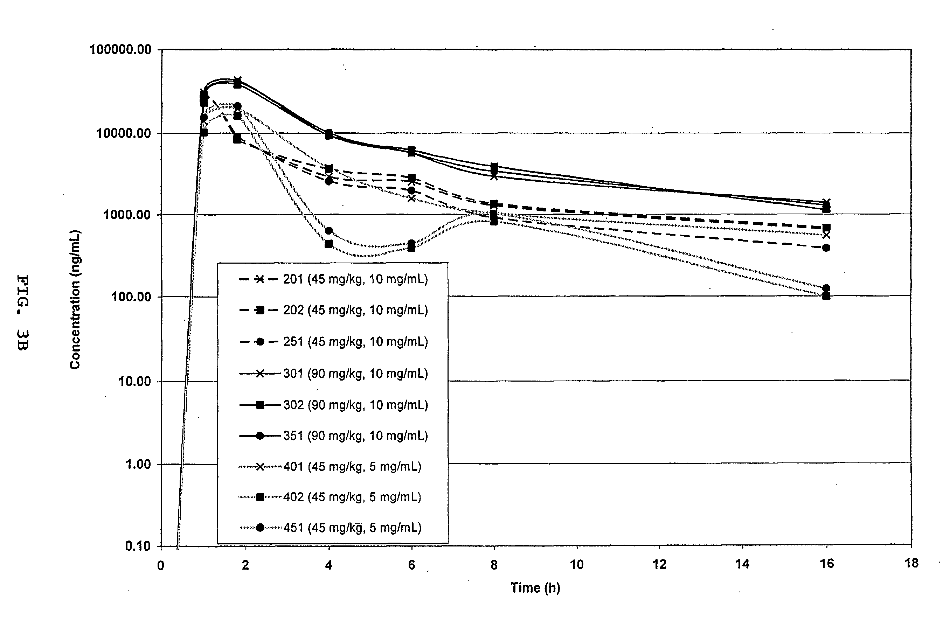

FIG. 3 comprises FIGS. 3A and 3B and is a graphical representation of the effect of concentration in dog serum over time during day 1 after one IV administration to dogs of M₄N in a formulation including 30% (w/v) and hydoxypropyl β-cyclodextrin ("HP-β-CD") and 25% (v/v) PEG 300. FIG. 3A uses on a non-logarithmic scale, and FIG. 3B uses a logarithmic scale of concentration.

### DETAILED DESCRIPTION OF THE PREFERRED EMBODIMENTS

The present invention provides for novel compositions, kits and methods for treatment of diseases, including proliferative diseases such as cancer and psoriasis, hypertension, obesity, type I or type II diabetes, central nervous system diseases or neurodegenerative diseases including, without limitation, pain, Alzheimer's disease, amyotrophic lateral sclerosis, Parkinson's disease, dementia, stroke, and inflammatory disease, premalignant neoplasia or dysplasia, infection including viral infections such as human immunodeficiency viruses ("HIV"), human T-cell lymphotropic virus ("HTLV"), human papilloma virus ("HPV"), herpes simplex viruses ("HSV"), hepatitis B virus ("HBV"), Epstein-Barr virus ("EBV"), Varicella-zoster, adenovirus, parvovirus, Jakob Creutzfeldt virus ("JC virus") or others.

The present invention provides for novel compositions containing the catecholic butanes including NDGA compounds, such as NDGA derivatives, for example M₄N, that are dissolved in certain pharmaceutically acceptable solubilizing agents, which together with other diluents, excipients and the like (collectively, "carrier") constitute formulations appropriate for injection into subjects, such as humans, for treatment of diseases. Such formulations are suitable for injection, such as intravenous administration. A suitable pharmaceutically acceptable carrier includes the water-soluble organic solvent polyethylene glycol ("PEG"), for example, PEG 300, PEG 400 or PEG 400 monolaurate and cyclodextrin or modified cyclodextrin such as hydroxypropyl-β-cyclodextrin ("HP-β-CD") or sulfobutyl ether β-cyclodextrin ("SBE-β-CD"). When PEG is used, it is used in the absence of ascorbic acid or BHT.

In one embodiment of the invention, the compounds herein are dissolved in PEG 300, PEG 400 or a PEG 400 monolaurate (the "PEG compounds"). Preferably, when PEG 400 is used, it is present in the absence of PEG 8000. In another embodiment, the compounds herein are dissolved in a modified cyclodextrin, such as HP-β-CD. In yet another embodiment, the present compounds are solubilized and diluted in a combination formulation containing one or more of the PEG compounds and HP-β-CD. For purposes herein, solubilization of the present compounds can be performed at room temperature or upon heating. Particularly useful are those solubilizers that maintain the present compounds in solution after cool-down when heat is applied in the solubilization process.

In addition, the present invention includes suitable materials for injection or infusion of the composition into an animal, such as intravenous ("IV") tubing, that is compatible for delivery of the present formulations. Suitable tubing include those made of polymers such as polytetrafluoroethylene ("PTFE") alone or in combination with a fluoroelastomer such as CHEM-Sure (Barnant Company), polyethylene, polypropylene, fluorinated ethylene propylene ("FEP"), Teflon® and platinum cured silicone (small size) (Cole-Parmer) and the like.

The present invention can be more clearly understood in light of the following definitions, which are used with other terms as defined elsewhere herein:

The term "active pharmaceutical ingredient," "API" or reference to the "compounds" as used herein means any of the catecholic butanes of the formula tetra-O-methyl NDGA.

"Alkylene dioxy" as used herein refers to methylene or substituted methylene dioxy or ethylene or substituted ethylene dioxy.

"Unsubstituted or substituted amino acid reside or salt thereof" in reference to one of the -R groups in Formula I or Formula II as used herein means an amino acid residue or a substituted amino acid residue including but not limited to: alanine, arginine, asparagine, aspartate, cysteine, glutamate, glutamine, glycine, histidine, isoleucine, leucine, lysine, methionine, phenylalanine, proline, serine, threonine, tryptophan, tyrosine, valine, 5-hydroxylysine, 4-hydroxyproline, thyroxine, 3-methylhistidine, ε-N-methyllysine, ε-N,N,N-trimethyllysine, aminoadipic acid, γ-carboxyglutamic acid, phosphoserine, phosphothreonine, phosphotyrosine, N-methylarginine, N-acetyllysine, and an N,N-dimethyl-substituted amino acid residue, or a salt of any of the foregoing, such as a chloride salt.

The "buffer" suitable for use herein includes any buffer conventional in the art, such as, for example, Tris, phosphate, imidazole, and bicarbonate.

A "carrier" as used herein refers to a non-toxic solid, semisolid or liquid filler, diluent, vehicle, excipient, solubilizing agent, encapsulating material or formulation auxiliary of any conventional type, and encompasses all of the components of the composition other than the active pharmaceutical ingredient. The carrier may contain additional agents such as wetting or emulsifying agents, or pH buffering agents. Other materials such as anti-oxidants, humectants, viscosity stabilizers, and similar agents may be added as necessary.

"Catecholic butane" as used herein means a compound of Formula I: 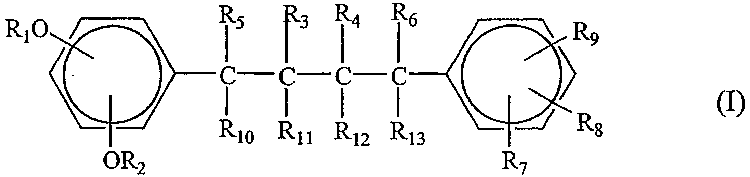 wherein R₁ and R₂ each independently represents -H, a lower alkyl, a lower acyl, an alkylene; or -R₁O and -R₂O each independently represents an unsubstituted or substituted amino acid residue or salt thereof; R₃, R₄, R₅, R₆, R₁₀, R₁₁, R₁₂ and R₁₃ each independently represents -H or a lower alkyl; and R₇, R₈, and R₉ each independently represents -H, -OH, a lower alkoxy, a lower acyloxy, an unsubstituted or substituted amino acid residue or a salt thereof, or any two adjacent groups together may be an alkyene dioxy.

A "cyclodextrin" as used herein means an unmodified cyclodextrin or a modified cyclodextrin, and includes with out limitation α-cyclodextrin, β-cyclodextrin, γ-cyclodextrin and any modified cyclodextrins containing modifications thereto, such as HP-β-CD or SBE-β-CD. Cyclodextrin typically has 6 (α-cyclodextrin), 7 (β-cyclodextrin), and 8 (γ-cyclodextrin) sugars, up to three substitutions per sugar, and 0 to 24 primary substitutions are therefore possible (primary substitutions are defined as substitutions connected directly to the cyclodextrin ring). The modified or unmodified cyclodextrins used in the present invention may have any appropriate number and location of primary substitutions or other modifications.

A "derivative" of NDGA as used herein means an "NDGA derivative" (see below).

The term "disease" as used herein includes all diseases, conditions, infections, syndromes or disorders for which the application of the present composition produces a therapeutic effect. Such "disease" includes, for example without limitation, cancer, psoriasis and other proliferative diseases, inflammatory disorders including rheumatoid arthritis, osteoarthritis, ulcerative colitis, Crohn's disease, atherosclerosis, chronic obstructive pulmonary disease ("COPD"), hypertension, obesity, diabetes, stroke and/or other neuronal disorders or neurodegenerative diseases or conditions, including Alzheimer's disease, Parkinson's disease, multiple sclerosis, amyotrophic lateral sclerosis ("ALS") and premalignant conditions such as intraepithelial neoplasia or dysplasia, and infectious diseases.

"G₄N" or "tetra-N,N-dimethyl glycinyl NDGA" or "tetra-dimethyl glycinyl NDGA" as used herein is an NDGA derivative of Formula II (below) in which R₁₄, R₁₅, R₁₆ and R₁₇ each independently represents -O(C=O)CH₂N(CH₃)₂ or -O(C=O)CH₂N⁺(CH₃)₂·Cl⁻, in either a solid form or in solution; and R₁₈ and R₁₉ each represents -CH₃.

"Lower acyl" as used herein means C₁- C₆ acyl, preferably, C₁ - C₃ acyl.

"Lower alkyl" as used herein means C₁- C₆ alkyl, preferably, C₁ - C₃ alkyl.

"M₄N" or "tetra-O-methyl NDGA" as used herein is an NDGA derivative of Formula II in which R₁₄, R₁₅, R₁₆ and R₁₇ each independently represents -OCH₃, and R₁₈ and R₁₉ are each -CH₃.

A "modified cellulose" as used herein means a cellulose that contains one or more modifications to the cellulose molecule and includes, for example EC, HPMC, CMC and MC.

"NDGA" as used herein means nordihydroguaiaretic acid and has the following formula:

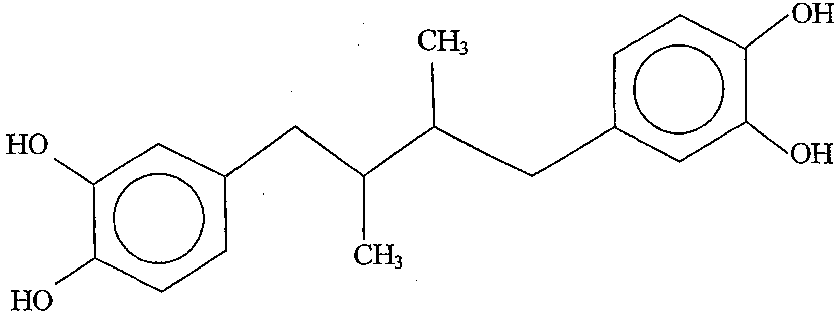

"NDGA compound" as used herein means singly or collectively NDGA and/or any one or more of the NDGA derivatives.

"NDGA derivative" as used herein means a derivative of NDGA having a Formula II 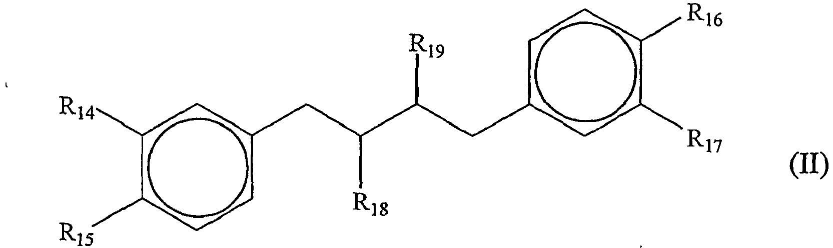 wherein R₁₄, R₁₅, R₁₆ and R₁₇ each independently represents -OH, a lower alkoxy, a lower acyloxy, or an unsubstituted or substituted amino acid residue or pharmaceutically acceptable salt thereof; and R₁₈ and R₁₉ each independently represents -H or a lower alkyl, wherein R₁₄, R₁₅, R₁₆ and R₁₇ are not simultaneously -OH. Thus, the term includes a compound that is a methylated derivative of NDGA, such as tetra-O-methyl NDGA (M₄N), tri-O-methyl NDGA (M₃N), di-O-methyl NDGA (M₂N) and mono-O-methyl NDGA (M₁N). Alternatively, a NDGA derivative may be a compound in which one or more of the hydrogens in the hydroxyl or methyl groups of NDGA are substituted, such as, for example where R₁₄, R₁₅, R₁₆ and R₁₇ each independently represents a lower alkoxy, a lower acyloxy, or an amino acid or substituted amino acid or salt thereof; and R₁₈ and R₁₉ each independently represents -H or an alkyl such as a lower alkyl. The term includes, for example, a compound in which R₁₄, R₁₅, R₁₆ and R₁₇ each independently represents -OCH₃ or -O(C=O)CH₃ or a disubstituted amino acid residue, such as a N,N-dimethyl substituted amino acid residue, such as -O(C=O)CH₂N(CH₃)₂ or -O(C=O)CH₂N⁺(CH₃)₂·Cl⁻; and R₁₈ and R₁₉ each represents -H or a lower alkyl, for example, -CH₃ or -CH₂CH₃.

As used herein, "percent," "percentage" or the symbol "%" means the percent of the component indicated in the composition based on the amount of the carrier present in the composition, on a weight/weight (w/w), weight/volume (w/v) or volume/volume (v/v), as indicated with respect to any particular component, all based on the amount of the carrier present in the composition. Thus, different types of carriers may be present in an amount of up to 100% as indicated, which does not preclude the presence of the API, the amount of which may be indicated as a % or as a certain number of mg present in the composition or a certain number of mg/mL present, where the % or mg/mL is based on the amount of the total carrier present in the composition. Certain types of carriers may be present in combination to make up 100% of the carrier.

A "pharmaceutically acceptable carrier" as used herein is non-toxic to recipients at the dosages and concentrations employed, and is compatible with other ingredients of the formulation. For example, the carrier for a formulation containing the present catecholic butane, preferably does not include oxidizing agents and other compounds that are known to be deleterious to such. A pharmaceutically acceptable carrier comprises a solubilizing agent. Suitable pharmaceutically acceptable carriers include, but are not limited to, water, dextrose, glycerol, saline, ethanol, buffer, Cremaphor® EL, phosphate buffered saline, PEG 300, PEG 400, modified cyclodextrin, and combinations thereof, all as set forth above.

The term "pharmaceutically acceptable excipient," includes vehicles, adjuvants, or diluents or other auxiliary substances, such as those conventional in the art, which are readily available to the public, and which are non-toxic to recipients at the dosages and concentrations employed, and is compatible with other ingredients of the formulation.. For example, pharmaceutically acceptable auxiliary substances include pH adjusting and buffering agents, tonicity adjusting agents, stabilizers, wetting agents and the like.

The term "solubilizing agent" as used herein means a composition in which one or more of the catecholic butanes or NDGA compounds, such as NDGA derivatives, dissolves. A solubilizing agent may also be a carrier or a pharmaceutically acceptable carrier.

The terms "subject," "host," and "patient," are used interchangeably herein to refer to an animal being treated with the present compositions, including, but not limited to, simians, humans, felines, canines, equines, bovines, porcines, ovines, caprines, mammalian farm animals, mammalian sport animals, and mammalian pets.

A "substantially purified" compound in reference to the catecholic butanes or NDGA compounds or derivatives for administration herein is one that is substantially free of materials that are not the catecholic butane, NDGA compounds or NDGA derivatives (hereafter, "non-NDGA materials"). By substantially free is meant at least about 50% free of non-NDGA materials, preferably at least about 70%, more preferably at least about 80%, even more preferably at least about 90% free and still more preferably at least about 95% free of non-NDGA materials.

As used herein, the terms "treatment," "treating," and the like, refer to obtaining a desired pharmacologic and/or physiologic effect. The effect may be prophylactic in terms of completely or partially preventing a condition or disease or symptom thereof and/or may be therapeutic in terms of a partial or complete cure for a condition or disease and/or adverse affect attributable to the condition or disease. "Treatment," thus, for example, covers any treatment of a condition or disease in a mammal, particularly in a human, and includes: (a) preventing the condition or disease from occurring in a subject which may be predisposed to the condition or disease but has not yet been diagnosed as having it; (b) inhibiting the condition or disease, such as, arresting its development; and (c) relieving, alleviating or ameliorating the condition or disease, such as, for example, causing regression of the condition or disease.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It must be noted that as used herein, the singular forms "a", "an", and "the" include plural referents unless the context clearly dictates otherwise. Thus, for example, reference to "a compound" includes a plurality of such compounds and reference to "the NDGA compound" includes reference to one or more NDGA compounds, such as NDGA derivatives, and equivalents thereof known to those skilled in the art.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. Citations to references mentioned in the text of this application are identified more fully in the bibliography preceding the claims.

The invention described below is given by way of example only and is not to be interpreted in any way as limiting the invention.

### Preparation of Catecholic Butanes

The catecholic butanes of the present invention can be prepared by any method know in the art. For example, such compounds can be made as described in US Patent 5,008,294.

### Preparation of the NDGA Derivatives

NDGA may be purchased from any available commercial sources, such as, for example, Alexis Biochemicals Corp., San Diego, CA, U.S.A. (Cat. No. LKT-N5669), or A.G. Scientific, Inc., San Diego, CA, U.S.A. (Cat. No. N1071), or Cayman Chemical Company, Ann Arbor, MI, U.S.A. (Cat. No. 70300).

The NDGA derivatives and formulations thereof can be made by any process conventional in the art. For example, the NDGA derivatives can be made as described in, US Patent 5,008,294; US Patent 6,291,524; Hwu, J.R. et al. (1998); or McDonald, R.W. et al. (2001).

In one embodiment of the present invention, an NDGA derivative, tetra-O-methyl NDGA, also known as meso-1,4-bis(3,4-dimethoxyphenyl)-2,3-dimethylbutane, or M₄N, is made as follows: a solution is made containing NDGA and potassium hydroxide in methanol in a reaction flask. Dimethyl sulfate is then added to the reaction flask and the reaction is allowed to proceed. The reaction is finally quenched with water, causing the product to precipitate. The precipitate is isolated by filtration and dried in a vacuum oven. The compound is then dissolved in a solution of methylene chloride and toluene and subsequently purified through an alumina column. The solvents are removed by rotary evaporation and the solid is resuspended in isopropanol and isolated by filtration. The filter cake is dried in a vacuum oven. The resulting tetra-O-methyl NDGA (M₄N) is crystallized by refluxing the filter cake in isopropanol and re-isolating the crystals by filtration.

In some embodiments of the present invention, certain NDGA derivatives of the present invention, such as G₄N, also known as meso-1,4-bis[3,4-(dimethylaminoacetoxy)phenyl]-(2R,3S)-dimethylbutane or tetra-dimethylglycinyl NDGA, or a hydrochloride salt thereof, and similar compounds having amino acid substituents, can also be prepared according to conventional methods, as described in, for example, US Patent 6,417,234.

### Preparation of the Therapeutic Compositions

The present invention provides compositions, including pharmaceutical compositions, comprising the catecholic butanes, as active pharmaceutical ingredients ("API"), and pharmaceutically acceptable carriers or excipients. Typically, the compositions of the instant invention will contain from less than 0.1% (w/v) up to 99% (w/v) of the active pharmaceutical ingredient or API, that is, the catecholic butanes, including the NDGA compounds and NDGA derivatives herein; optionally, the present invention will contain 2% (w/v) to 90% (w/v) of the API.

The present invention additionally provides compositions in which the catecholic butanes, for example M₄N, are present in concentrations of about 1 mg/mL to about 200 mg/mL, or about 10 mg/mL to about 175 mg/mL, or about 20 mg/mL to about 150 mg/mL, or about 30 mg/mL to about 125 mg/mL, or about 40 mg/mL to about 100 mg/mL, or about 50 mg/mL to about 75 mg/mL. In one embodiment, the NDGA compounds are present in the compositions herein at a concentration about 1 mg/mL, about 2 mg/mL, about 2.5 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 75 mg/mL, about 80 mg/mL, about 90 mg/mL, about 100 mg/mL, about 120 mg/mL, about 125 mg/mL, about 150 mg/mL, about 175 mg/mL or about 200mg/mL.

Expressed alternatively, other embodiments of the composition of the present invention may contain less than about 0.1 mg to about 200 mg or more of the API, such as about 10 mg, about 20 mg, about 25 mg, about 30 mg, about 40 mg, about 50 mg, about 60 mg, about 75 mg, about 100 mg or about 200 mg of the API.

The pharmaceutically acceptable carrier or excipient may contain one or more solubilizing agents in which the API is dissolved. The pharmaceutically acceptable carrier or excipient may additionally contain a diluent.

In one embodiment, the invention provides a solubilizing agent that contains one or more of a water-soluble organic solvent, other than DMSO. Among preferred water-soluble organic solvents are PEG compounds such as: PEG 300, PEG 400. The PEG compound in the present compositions is provided in an amount of 5% to 100%, or 5% to 60%, or 10% to 90%, or 20% to 80%, or 30% to 70%, or 40% to 60%, all concentrations being a percentage of volume/volume (v/v).

The concentration of the PEG compounds in the present compositions can vary depending on what other solubilizers or diluents or excipients are also present. For example, the PEG 300 or PEG 400 of the present invention can be at a concentration of 5%, 10%, 12.5%, 15%, 20%, 25%, 30%, 35%, 40%, 45%, 50%, 55%, 60%, 65%, about 70%, 75%, 80%, 85%, 90%, or 95%, all such concentrations being given as a percentage of volume/volume (v/v).

The present invention also provides compositions of M4N compounds in a cyclodextrin, which includes modified cyclodextrins. The cyclodextrins herein may be α-cyclodextrin, β-cyclodextrin, γ-cyclodextrin, and the modified cyclodextrins may include HP-β-CD and SBE-β-CD, for example. In one embodiment, the present composition contains a modified cyclodextrin in a concentration of 5% to 80%, or 10% to 70%, or 20% to 60%, or 30% to 50%, all such concentrations being given as a percentage of weight/volume (w/v).

In yet another embodiment, the modified cyclodextrins, such as HP-β-CD, is present in the compositions at a concentration of 12.5%, 15%, 20%, 25%, 30%, 35%, 40%, 45%, 50%, 55%, 60%, 65%, 70% or 75%, all such concentrations being given as a percentage of weight/volume (w/v).

Another pharmaceutically acceptable carrier or excipient that may be used alone or with others in the composition of the present invention is an ionic, non-ionic or amphipathic surfactant, such as Cremophor® EL, polysorbates, which are non-ionic surfactants, for example, polysorbate 20 and polysorbate 80, commercially available as Tween® 20 or Tween® 80, TPGS, which is an amphipathic surfactant, among many others. Further examples of suitable surfactants include, without limitation, glycerol monooleate and an esterified fatty acid, such as those typically made by transesterification of vegetable oils, available in several varieties and grades as Labrafil®, Labrasol®, and Gelucire®, from Gattefosse Corp., Paramus, NJ, U.S.A. The surfactant can be present in any desired effective amount, such as at a concentration of about 1% (v/v) to about 100% (v/v), preferably about 9% (v/v) to about 80% (v/v), and more preferably, about 10% (v/v) to about 50% (v/v). As specific examples, preferred concentrations of a non-ionic surfactant are Tween® 20 at a concentration of about 9% (v/v) to about 100% (v/v) and Tween® 80 of about 33% (v/v) to about 100% (v/v). All percentages of the surfactant are volume percentages (v/v).

Another pharmaceutically acceptable carrier or excipient that may be used alone or with others in the composition of the present invention is a modified cellulose, such as EC, HPMC, MC and CMC. The modified cellulose can be present in any desired effective amount, such as a concentration of about 0.1% to about 25%, or about 0.5% to about 7.5%, or about 1.0% to about 5%. As specific examples, EC may be present at a concentration of about 5% to about 20%; HPMC may be present at a concentration of about 0.5% to about 1%; MC may be present at a concentration of about 1% to about 3%; and CMC may be present at a concentration of about 1% to about 4%. The percentages of modified cellulose are in weight per volume (w/v).

Another pharmaceutically acceptable carrier or excipient that may be used alone or with others in the composition of the present invention is a water-insoluble lipid, such as an oil or mixed fat emulsion. Examples of oils include corn oil, sesame seed oil, peppermint oil, soybean oil, mineral oil and glycerol, for instance. Mixed fat emulsion compositions are available, such as Intralipid® emulsion, as described above.

The water-insoluble carriers may be used in combination with any one or more of the water-soluble carriers, such as PEG compounds and the surfactants, such as Tween® 20 or Tween® 80.

The water-insoluble lipid carriers can be present in any desired effective amount, such as a concentration of about 10% (v/v) to about 100% (v/v), or about 15% (v/v) to about 85% (v/v), or about 25% (v/v) to about 75% (v/v). Oil may be present at a concentration of about 9% (v/v) to about 100% (v/v). Mixed fat emulsions may be present at a concentration of about 10% (w/v) to about 30% (w/v); and preferably about 20% (w/v).

Combinations of the various carrier components may be used with the API, as noted above. One non-limiting example of such an embodiment that is presently preferred is a composition of 10 mg/ml M₄N in 25% (w/v) PEG 300, 30% (w/v) HP-β-CD, balance of the carrier being water suitable for injection into animals ("WFI," which designates a recognized grade of water in the pharmaceutical industry). In this preferred embodiment, the HP-β-CD has 6 to 8 degrees of substitution, but other substitutions in other embodiments are well within the scope of this invention, as noted above.

It is to be understood that as long as the catecholic butanes herein are dissolved and remain in solution, one or more of the solubilizers or diluents or excipients herein may be added to such solution to optimize delivery of such to a subject in need of such treatment.

In another embodiment, the invention provides a diluent that is saline or water that is suitable for injection. In one aspect of the invention, when the osmolarity of the pharmaceutical composition is high, water suitable for injection is used as a diluent.

Pharmaceutically acceptable carriers or excipients suitable for use herein are described in a variety of publications. Examples of useful carriers or excipients are described in, for example, Gennaro, A.R. (2003); Ansel, H.C. et al. (2004); Rowe, R.C. et al. (2003); and Garg, S. et al. (2001).

The compositions in liquid form may include a buffer, which is selected according to the desired use of the catecholic butanes and may also include other substances appropriate for the intended use. Those skilled in the art can readily select an appropriate buffer, a wide variety of which are known in the art, suitable for an intended use.

### Therapeutic Methods

The compositions containing the catecholic butanes, find use as therapeutic agents or for treatment in subjects in need of such treatment in any number of diseases in which such catecholic butanes can be used.

The present invention provides for methods and compositions for treatment of disease including, for example, proliferative diseases such as benign and malignant cancer, psoriasis and premalignant conditions and neoplasia, such as intraepithelial neoplasia, or dysplasia. The present invention also provides for treatment of diabetes, including type I and type II diabetes, obesity and complications resulting from such, including cardiovascular diseases, stroke and hypertension. The present invention further provides for treatment of inflammatory diseases including rheumatoid arthritis, osteoarthritis, multiple sclerosis, ulcerative colitis, Crohn's disease, chronic obstructive pulmonary disease (COPD) and other immune system associated diseases. Additionally, the present invention provides for treatment of neurological diseases, including central nervous system diseases and neurodegenerative diseases such as Alzheimer's disease, dementia, amyotrophic lateral sclerosis and Parkinson's disease. In a further embodiment, the present invention provides for treatment of infections, such as viral infections including viruses that require Sp1 binding for transcription or replication. Examples of such viruses that require Sp1 binding include: HIV, HTLV, HPV, HSV, HBV, EBV, Varicella-zoster virus, adenovirus, parvovirus and JC virus.

A variety of animal hosts are treatable according to the subject methods, including human and non-human animals. Generally such hosts are "mammals" or "mammalian," where these terms are used broadly to describe organisms which are within the class mammalia, including the orders carnivore (*e.g*., dogs and cats), rodentia (*e.g*., guinea pigs, and rats), and other mammals, including cattle, goats, horses, sheep, rabbits, pigs, and primates (*e.g*., humans, chimpanzees, and monkeys). In many embodiments, the hosts will be humans. Animal models are of interest for experimental investigations, such as providing a model for treatment of human disease. Further, the present invention is applicable to veterinary care as well.

### Formulations, dosages, and routes of administration

In one embodiment of the invention, an effective amount of the present composition is administered to the host, where an "effective amount" means a dosage sufficient to produce a desired result. In some embodiments, the desired result is at least an inhibition of progression of the neoplasia or dysplasia.

The appropriate dose to be administered depends on the subject to be treated, such as the general health of the subject, the age of the subject, the state of the disease or condition, the weight of the subject, the size of the tumor, for example. Generally, about 0.1 mg to about 500 mg or less may be administered to a child and about 0.1 mg to about 5 grams or less may be administered to an adult. Typical dosages are within the broad range of about 10 mg of active pharmaceutical ingredient per kg weight of the subject to about 600 mg of active pharmaceutical ingredient per kg weight of the subject. The active agent can be administered in a single or, more typically, multiple doses. Preferred dosages for a given agent are readily determinable by those of skill in the art by a variety of means. Other effective dosages can be readily determined by one of ordinary skill in the art through routine trials establishing dose response curves. The amount of agent will, of course, vary depending upon the particular agent used.

The frequency of administration of the active agent, as with the doses, will be determined by the care giver based on age, weight, disease status, health status and patient responsiveness. Thus, the agents may be administered one or more times daily, weekly, monthly or as appropriate as conventionally determined. The agents may be administered intermittently, such as for a period of days, weeks or months, then not again until some time has passed, such as 3 or 6 months, and then administered again for a period of days, weeks, or months.

In pharmaceutical dosage forms, the active agents may be administered alone or in appropriate association, as well as in combination, with other pharmaceutically active agents or therapeutics including other small molecules, antibodies or protein therapeutics. The following methods and excipients are merely exemplary and are in no way limiting.

In addition, if desired, the carrier or excipient may contain minor amounts of auxiliary substances such as pH adjusting and buffering agents, tonicity adjusting agents, stabilizers, wetting agents or emulsifying agents. Actual methods of preparing such dosage forms are known, or will be apparent, to those skilled in the art. *See, e.g.,* Remington's Pharmaceutical Sciences, 20th ed., Mack Publishing Co. Rawlins EA, (1997). The composition or formulation to be administered will, in any event, contain a quantity of the API adequate to achieve the desired state in the subject being treated.

Unit dosage forms for injection or intravenous administration may comprise the API in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of API of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

Kits with multiple or unit doses of the active agent are included in the present invention. In such kits, in addition to the containers holding the multiple or unit doses of the compositions containing the catecholic butanes such as the NDGA compounds will be an informational package insert with instructions describing the use and attendant benefits of the drugs in treating pathological condition of interest. Optionally, an applicator for administration of the present composition is included in each kit.

The present injectable compositions can be administered parenterally, including intravenously, intra-arterially, intraperitoneally, subcutaneously and intravesicularly, as appropriate for the disease to be treated and as conventional in the art. While the compositions of the present invention are intended to be administered by injection, they may also be suitable to be administered by other routes, for example by topical, intranasal, inhalation or implantation administration.

The Examples set forth below are exemplary in nature and are not to be interpreted as limiting the present invention.

### Example 1. Formulations Containing M₄N in HP-β-CD and/or PEG 300

In this example, M₄N was prepared as described in PCT/US2004/016117 and was solubilized in a solubilizing agent. The resulting solution was optionally mixed with an excipient and/or a diluent. The solubilizing agent and the excipient may be used interchangeably or in combination with each other. One solubilizing agent or excipient used was endotoxin-controlled hydroxypropyl-β-cyclodextrin ("HP-β-CD") obtained from Research Diagnostics, Inc. (Cat. No. RDI-82004HPB, lot no. H3N188P) (Flanders, NJ, U.S.A.). Another solubilizing agent or excipient used was PEG 300, obtained from Spectrum Chemicals, Inc. (Cat. No. P0108, lot no. TB1228) (Gardena, CA, U.S.A.).

In one embodiment of the invention, HP-β-CD and PEG 300 were present in a single formulation. To make this formulation, M₄N was first dissolved in PEG 300 to form a M₄N in PEG 300 solution ("M₄N/PEG 300"). The M₄N/PEG 300 solution was then added to a pre-made solution of HP-β-CD to form a M₄N solution in a PEG 300 and HP-β-CD (hereafter, a "CPE" formulation").

When preparing the HP-β-CD solution, a volume expansion must be accounted for. For example, for a 40% (w/v) HP-β-CD solution, a volume expansion of 0.7 mL/g (i.e., 0.7 mL of water displaced per gram of HP-β-CD added) must be accounted for.

A 100 mL solution of 40% HP-β-CD for use as a solubilizing agent and/or excipient was made as follows: 65 mL of WFI were placed in a glass beaker containing a stir bar. The beaker was placed on a magnetic plate, and the stir bar was set to stir at medium speed. About forty (40) grams of HP-β-CD were added slowly to the stirring WFI, using a spatula to direct the HP-β-CD to the center of the beaker so as to prevent HP-β-CD crystals from sticking to the beaker wall. The HP-β-CD solution was stirred for about 24 hr or until the HP-β-CD was dissolved completely upon visual inspection. The resulting solution measured about 93 mL. About 7 mL of WFI was added to this resulting solution to obtain 100 mL, producing a final solution of about 40% HP-β-CD. The final solution was stirred for about 1 hr, stored at room temperature, and was protected from light. This method of making the modified cyclodextrin solution may be scaled up or down to obtain the desired volume or concentration. Other concentrations or other modified cyclodextrin solutions may be similarly made, for example, by substituting HP-β-CD with other modified cyclodextrins, adjusted for the appropriate concentrations in the process described above.

A 10 mL solution of M4N at a concentration of about 10 mg/mL in 40% HP-β-CD was made as follows. About 10 mL of the 40% HP-β-CD solution were added to a glass beaker containing a stir bar. The beaker was placed on a magnetic plate and the stir bar was set to stir at medium speed. About 10 mg of M₄N were slowly added to the 40% HP-β-CD in the center of the beaker with the aid of a spatula. The M₄N/40% HP-β-CD mixture was stirred for 2 hr or until all M₄N was uniformly suspended without any clumps being present. The M₄N/40% HP-β-CD mixture was optionally heated at 80°C for about 30 min. (or longer if a larger volume of solution was desired, for example, 1 hr at 80° C for 100 mL of the M₄N/HP-β-CD mixture), or longer as needed to ensure complete dissolution of M₄N. The M₄N/HP-β-CD mixture or solution was observed for presence of any undissolved M₄N by holding the beaker against a white background followed by a dark background, looking for presence of particulates. The final M₄N/40% HP-β-CD solution was stored at room temperature and was kept protected from light. This process may be scaled up or down to obtain the requisite volume or concentration of M₄N. Formulations containing M₄N in other cyclodextrin solutions may be similarly made, such as, for example, by substituting HP-β-CD in the process described above with other cyclodextrins. Results shown in Table 1 demonstrate that M₄N remained in solution after cooling at concentrations of 1 mg/mL and 10 mg/mL in the 40% HP-β-CD formulation for greater than 7 days.

A 100 mL solution of M₄N at a concentration of about 25 mg/mL in PEG 300 was made as follows. About 100 mL of PEG 300 were added to a glass beaker containing a stir bar. The beaker was placed on a magnetic plate and the stir bar was set to stir at medium speed. About 2.5 g of M₄N were slowly added to the PEG 300 in the center of the beaker with the aid of a spatula to prevent M₄N from sticking to the beaker wall. The M₄N/PEG 300 mixture was stirred for 24 hr or until all M₄N had dissolved or was uniformly suspended without any clumps being present. The M₄N/PEG 300 mixture was optionally heated at about 60° C for about 30 min. (or longer if a larger volume of solution was desired, for example, 1 hr at 60° C for 500 mL of the M₄N/PEG 300 mixture), or longer as needed to ensure complete dissolution of M₄N. The M₄N/PEG 300 mixture or solution was observed for presence of any undissolved M₄N by holding the beaker against a white background followed by a dark background, looking for presence of particulates. After all M₄N was observed to be dissolved, the resulting M₄N/PEG 300 solution was used immediately or before the expiration of 48 hr, otherwise crystals or other precipitates might form. If crystals formed, the M₄N /PEG 300 solution could be heated again at 60° C for about 1 hr, with stirring, on a hot magnetic plate until all M₄N was dissolved back in solution. The final M₄N/PEG 300 solution was stored at room temperature and was kept protected from light. This process may be scaled up or down to obtain the requisite volume or concentration of M₄N. Formulations containing M₄N in other PEGs may be similarly made, such as, for example, by substituting PEG 300 in the process described above with PEG 400 or PEG 400 monolaurate.

A 100 mL stock solution of a formulation containing 50% PEG 300 (v/v), 20% HP-β-CD (w/v) and 12.5 mg M₄N was made by adding 50 mL of the 40% pre-made HP-β-CD solution (made as described above) to a glass beaker containing a stir bar on a magnetic plate, with the stir bar stirring at medium speed, and slowly adding 50 mL of the pre-made M₄N/PEG 300 solution (made as described above), for example, at a rate of about 10 mL per min. The M₄N/PEG 300 was added by use of a pipette to the center of the beaker to avoid its sticking to the walls of the beaker and to ensure complete dissolution. The addition of M4N/PEG 300 to the HP-β-CD solution initially appeared as a white solution, but eventually became clear upon continuous mixing. This recipe may be scaled up or down as appropriate to produce the desired volume or concentration of M₄N/PEG 300 and HP-β-CD. The stock solution was filter-sterilized using a 0.22 µm PVDF membrane, such as a pre-sterilized vacuum driven disposal bottle-top filter membrane obtained from Millipore (Cat. No. SCGV T05 RE) (Billerica, MA, U.S.A.). The filtration process was driven by vacuum force and the filtrate is collected in pre-sterilized 250 mL glass bottles. The bottles were then sealed tightly, stored at room temperature and protected from light. A stock solution of M₄N/PEG 400 or M₄N/PEG 400 monolaurate in HP-β-CD can be similarly made by substituting PEG 300 with PEG 400 or PEG 400 monolaurate in the processes described above.

The M₄N/PEG 300/HP-β-CD, M₄N/PEG 400/HP-β-CD or M₄N/PEG 400 monolaurate/HP-β-CD stock solution made in the foregoing manner can be diluted prior to use *in vitro* or for administration into animals. If dilution is necessary, the stock solution is preferably diluted in WFI, instead of saline, for example, so as to keep the osmolarity down. To make a 100 mL of a 1:1 dilution of the stock solution in WFI, about 50 mL of the stock solution was added to a glass vial. About 50 mL of WFI was added to the 50 mL of the stock solution in the vial to form a diluted solution. The glass vial was closed and the diluted solution was mixed well by shaking and inverting the vial a few times. The diluted solution was filter-sterilized using a 0.22 µm PVDF membrane, such as a pre-sterilized vacuum driven disposal bottle-top filter membrane obtained from Millipore (Cat. No. SCGV T05 RE) (Billerica, MA, U.S.A.). The filtration process was driven by vacuum force and the filtrate was collected in pre-sterilized 250 mL glass bottles. The bottles were then sealed tightly, stored at room temperature and protected from light. This process may be scaled up or down to obtain the requisite volume or dilutions, such as 1:2 or 1:4 dilutions, for example.

A formulation suitable for use as placebo control containing 50% PEG 300 and 20% HP-β-CD can be made as follows. To make a 100 mL solution of the placebo or control formulation, about 50 mL of 40% HP-β-CD are added to a glass beaker containing a stir bar on a magnetic plate. The magnetic plate is set to stir the HP-β-CD solution at medium speed. About 50 mL of PEG 300 are slowly added to the 50 mL of HP-β-CD in the glass beaker by pipette to the center of the beaker to prevent the PEG 300 from sticking to the wall of the beaker. The mixture is stirred for about 1 hr or until mixing is complete. This placebo formulation is filter-sterilized using a 0.22 µm PVDF membrane filter driven by vacuum force. The filtrate is collected in pre-sterilized 250 mL glass bottles. The glass bottles are sealed tightly, stored at room temperature, and kept protected from light. This recipe may be scaled up or down as required to produce the desired concentration and volume amounts. Further, PEG 300 may be substituted with PEG 400 or PEG 400 monolaurate, as desired.

Results of the solubility of M₄N in formulations containing HP-β-CD and/or PEG 300, PEG 400, and in formulations containing HP-β-CD and propylene glycol ("PG"), hydroxypropyl methylcellulose (HPMC), carboxyl methylcellulose (CMC), polyvinyl pyrrolidone (PVP), or Tween® 80 made in accordance to the foregoing or similar processes as well as characteristics of the resulting formulations are shown in Tables 1 - 5, where N represents "No" and Y represents "Yes."

**Table 1. Modified Cyclodextrins as Solubilizing Agents and/or Excipients**

| Excipients | Excipient Concentration (in w/v unless otherwise specified) | Drug Concentration (in mg/mL unless otherwise specified) | Dissolution After Rotation | Dissolution After Heating at X°C | Dissolution After Cool Down | Stability at Room Temperature |
|---|---|---|---|---|---|---|
| α-CD | 15% | 1 | N | N at 90° | | |
| | | 10 | N | N at 90° | | |
| | | 50 | N | N at 90° | | |
| | | 100 | N | N at 90° | | |
| | | | | | | |
| β-CD | 1.50% | 1 | N | N at 90° | | |
| | | 10 | N | N at 90° | | |
| | | | | | | |
| γ-CD | 5% | 1 | N | N at 90° | | |
| | | | | | | |
| HP-β-CD | 50% | 1 | N | Y at 90° | Y | > 7 days |
| | | 10 | N | Y at 90° | Y | > 7 days |
| | | 20 | N | N at 90° | | |
| | | 50 | N | N at 90° | | |
| | | 100 | N | N at 90° | | |
| | 40% | 1 | N | Y at 80° | Y | > 7 days |
| | | 10 | N | Y at 80° | Y | > 7 days |
| | | 12 | N | N at 80° | | |
| | | 14 | N | N at 80° | | |
| | | 16 | N | N at 80° | | |
| | | 20 | N | N at 80° | | |
| | | 50 | N | N at 80° | | |
| | | 100 | N | N at 80° | | |
| | 30% | 1 | N | Y at 90° | Y | > 7 days |
| | | 10 | N | Y at 90° | Y | < 3 days |
| | | 20 | N | N at 90° | | |
| | | 50 | N | N at 90° | | |
| | | 100 | N | N at 90° | | |
| | 20% | 1 | N | Y at 90° | Y | > 7 days |
| | | 10 | N | N at 90° | | |
| | 81.5% (w/w), Lyophilized | 185 mg/g | powder | | | |
| HP-β-CD | 50% in saline | 10 | N | Y at 90° | Y | >7 days |
| | 20% in saline | 1 | N | | | |
| | | 10 | N | | | |
| | | 50 | N | | | |
| | | | | | | |
| HP-β-CD and Propylene Glycol (PG) | 40% HP-β-CD, 2.5% PG (v/v) | 1 | N | N at 80° | | |
| | | 10 | N | N at 80° | | |
| | | | | | | |
| HP-β-CD and PVP | 50% HP-β-CD, 1.25% PVP (w/v) | 1 | N | Y at 90° | Y | < 3 days |
| | | 10 | N | N | | |
| | | 50 | N | N | | |
| | 40% HP-β-CD, 1% PVP (w/v) | 1 | N | N | | |
| | | 10 | N | N | | |
| | | | | | | |
| HP-β-CD and PEG 300 | 27% HP-β-CD, 33% PEG 300 (v/v) | 13.3 | N | Y at 60° | Y | > 7 days |
| | 23% HP-β-CD, 43 PEG 300 (v/v) | 12.9 | N | Y at 60° | Y | > 7 days |
| | 20% HP-β-CD, 50% PEG 300 (v/v) | 12.5 | N | Y at 60° | Y | > 7 days |
| | 15% HP-β-CD, 50% PEG 300 (v/v) | 12.5 | N | Y at 60° | Y | < 7 days |
| | 12.5% HP-β-CD, 50% PEG 300 (v/v) | 12.5 | N | Y at 60° | Y | < 1 day |
| | 13% HP-β-CD, 67% PEG 300 (v/v) | 16.7 | N | Y at 60° | N | |
| | 10% HP-β-CD, 77% PEG 300 (v/v) | 19.3 | N | Y at 60° | N | |
| | 10% HP-β-CD, 25% PEG 300 (v/v) | 6.25 | N | Y at 60° | Y | > 7 days |
| | 6.7% HP-β-CD, 16.7% PEG 300 (v/v) | 4.17 | N | Y at 60° | Y | > 7 days |
| | 5% HP-β-CD, 12.5% PEG 300 (v/v) | 3.13 | N | Y at 60° | Y | < 7 days |
| | | | | | | |
| HP-β-CD and PEG 400 | 32% HP-β-CD, 20% PEG 400 (v/v) | 10 | N | Y at 60° | Y | > 7 days |
| | 30% HP-β-CD, 25% PEG 400 (v/v) | 12.5 | N | Y at 60° | Y | > 7 days |
| | 27% HP-β-CD, 33% PEG 400 (v/v) | 13.3 | N | Y at 60° | Y | > 7 days |
| | 23% HP-β-CD, 43% PEG 400 (v/v) | 12.9 | N | Yat60°C | Y | > 7 days |
| | 20% HP-β-CD, 50% PEG 400 (v/v) | 12.5 | N | Y at 60° | Y | < 7 days |
| | 15% HP-β-CD, 50% PEG 400 (v/v) | 12.5 | N | Y at 60° | Y | < 3 days |
| | 12.5% HP-β-CD, 50% PEG 400 (v/v) | 12.5 | N | Y at 60° | Y | < 1 day |
| | 40% HP-β-CD, 5% PEG 400 (v/v) | 10 | N | Y at 60° | N | |
| | | | | | | |
| HP-β-CD and Tween® 80 | 27% HP-β-CD, 33% Tween® 80 (v/v) | 13.3 | N | Y at 60° | N | |

All formulations withstand 4°C for 24 hr and 5 min. centrifugation at 5000 rpm without forming visible precipitates. The formulation containing 50% PEG 300, 20% HP-β-CD, 12.5 mg/mL M₄N stock solution withstands 4°C for at least 4 months. Dilutions of the same stock made in 1:1 or 1:2 dilutions also withstand 4°C for at least 4 months.

**Table 2. Formulations of M₄N in PEG 300 and HP-β-CD**

| Undiluted Stock Solutions | | | For each 10 mg of M₄N | | For each 50 mg of M₄N | | For each 100 mg of M₄N | |
|---|---|---|---|---|---|---|---|---|
| PEG 300 (v/v) | HP-β-CD (w/v) | M₄N (mg/mL) | PEG 300 in mL (mg) | HP-β-CD (mg) | PEG 300 in mL (mg) | HP-β-CD (mg) | PEG 300 in mL (mg) | HP-β-CD (mg) |
| 50% | 15% | 12.5 | 0.4 (450) | 120 | 2.0 (2250) | 600 | 4.0 (4500) | 1200 |
| 50% | 20% | 12.5 | 0.4 (450) | 160 | 2.0 (2250) | 800 | 4.0 (4500) | 1600 |
| 43% | 23% | 12.9 | 0.33 (375) | 178 | 1.67 (1875) | 890 | 3.33 (3746) | 1780 |
| 33% | 27% | 13.3 | 0.25 (281) | 200 | 1.25 (1406) | 1000 | 2.5 (2813) | 2000 |

**Table 3. Formulations of M₄N in PEG 400 and HP-β-CD**

| Undiluted Stock Solutions | | | For each 10 mg of M₄N | | For each 50 mg of M₄N | | For each 100 mg of M₄N | |
|---|---|---|---|---|---|---|---|---|
| PEG 400 (v/v) | HP-β-CD (w/v) | M₄N (mg/mL) | PEG 400 in mL (mg) | HP-β-CD (mg) | PEG 400 in mL (mg) | HP-β-CD (mg) | PEG 400 in mL (mg) | HP-β-CD (mg) |
| 50% | 20% | 12.5 | 0.4 (450) | 160 | 2.0 (2250) | 800 | 4.0 (4500) | 1600 |
| 43% | 23% | 12.9 | 0.33 mL (375) | 178 | 1.67 (1875) | 890 | 3.33 (3746) | 1780 |
| 33% | 27% | 13.3 | 0.25 (281) | 200 | 1.25 (1406) | 1000 | 2.5 (2813) | 2000 |
| 25% | 30% | 12.5 | 0.20 (225) | 240 | 1.0 (1125) | 1200 | 2.0 (2250) | 2400 |
| 20% | 32% | 10.0 | 0.20 (225) | 320 | 1.0 (1125) | 1600 | 2.0 (2250) | 3200 |

**Table 4. Stability of M₄N formulations in PEG 300**

| Formulation | PEG 300 (v/v) | HP-β-CD (w/v) | M₄N (mg/mL) | Stability at Room Temperature |
|---|---|---|---|---|
| A | 50% | 15% | 12.5 | > 3 days |
| B | 50% | 20% | 12.5 | > 5 months |
| C | 43% | 23% | 12.9 | > 5 months |
| D | 33% | 27% | 13.3 | > 5 months |

**Table 5. Stability of M₄N formulations in PEG 400**

| Formulation | PEG 400 (v/v) | HP-β-CD (w/v) | M₄N (mg/mL) | Stability at Room Temperature |
|---|---|---|---|---|
| E | 50% | 20% | 12.5 | > 6 days |
| F | 43% | 23% | 12.9 | > 5 months |
| G | 33% | 27% | 13.3 | > 5 months |
| H | 25% | 30% | 12.5 | > 5 months |
| I | 20% | 32% | 10.0 | > 5 months |

Similarly, M₄N may be solubilized in other solubilizing agents, such as water-soluble organic solvents including ethanol, polyvinyl pyrrolidone (PVP), propylene glycol or glycerol.

Example 2. Effect of M₄N in DMSO or Combination PEG 300/ HP-β-CD Formulation on Proliferation and Death of Tumor Cells in Culture

M₄N in 10% (w/v) HP-β-CD and 25% (v/v) PEG 300 (hereafter, the "CPE formulation"), M₄N in 30% (w/v) HP-β-CD and 25% (v/v) PEG 300 (hereafter, the "CPE 25/30 formulation") and M4N in 27% (w/v) HP-β-CD and 33% (v/v) PEG 300 (hereafter, the "CPE 33/27 formulation") was tested for their effects on cell death and proliferation on two different tumor cell lines: HeLa, an HPV-18 positive human cervical cancer cell line, and C-33A, an HPV-negative human cervical cancer cell line M₄N in DMSO was also tested in parallel. Both tumor cell lines were treated with increasing amounts of M₄N: 0 µM, 20 µM, 40 µM, 60 µM and 80 µM, for 72 hr with the DMSO or the CPE formulation. Each formulation was added to total 1% of the growth media (Minimal Essential Medium with L-glutamine supplemented with 10% fetal bovine serum, 1mM sodium pyruvate, 1x non-essential amino acid solution, and 1,000 IU/mL penicillin/1,000 µg/mL streptomycin solution). Control cells were grown under the same conditions and were left untreated. After 72 hr of treatment or no treatment, the total number of cells and the number of live cells in each sample were counted, using the trypan blue exclusion method. The cell proliferation rate and the percentage of dead cells in each sample were analyzed. Results of this experiment are shown in FIG. 1, FIG. 2, and Tables 6 through 12.

FIG. 1 is a graphical representation of the ratio of the number of treated cells/number of untreated cells plotted against increasing concentrations of M₄N in either the DMSO formulation or the CPE formulation for treatment of the C-33A cells and HeLa cells. FIG. 2 is a graphical representation of the percentage of dead cells plotted against increasing concentrations of M₄N and in either DMSO formulation or the CPE formulation for treatment of the two cancer cell lines, C-33A and HeLa cells in culture.

Results show that DMSO alone, in the absence of M₄N, has a significant anti-proliferative effect and some toxic effect, as measured by % of dead cells, on both of the tumor cell lines tested as compared to the untreated controls. In contrast, the CPE formulation alone has an anti-proliferative effect and very little toxic effect on the two tumor cell lines when compared to the untreated controls.

Cell proliferation rate was reduced in both cell lines after treatment with M₄N in either the DMSO formulation or the CPE formulation, when compared to the non-M₄N-treated controls (i.e., 0 µg/mL or 0 µM of M₄N) in the same formulation. In fact, the anti-proliferative effect appeared to be M₄N dose-dependent in the CPE formulation. In the CPE formulation, for example, about 20 µM or 7.2 µg/mL of M₄N was found to be sufficient to cause about a 50% inhibition in cell proliferation for both tumor cell types. Further increases in the concentration of M₄N in the CPE formulation resulted in further increases in anti-proliferative effect for both cell types.

In general, higher doses M₄N in either the DMSO formulation or the CPE formulation induced higher percentages of cell death for both the C-33A cells and the HeLa cells. However, M₄N in the DMSO formulation was more toxic to cells than the corresponding concentrations of M₄N in the CPE formulation. While the highest concentration of M₄N tested (80 µM or 28.7 µg/mL) in DMSO formulation promoted cell death in about 40% of the cell population, the same concentration of M₄N in the CPE formulation promoted cell death in only about 20% of the cell population in this experiment.

These results were found to be reproducible in both cell lines. Data from this study indicate that M₄N in the CPE formulation has the ability to arrest cell proliferation, similar to M₄N in the DMSO formulation, while inducing less cellular toxicity than the DMSO formulation.

Data were collected from continuing time points to test the effectiveness of the CPE formulation over time. The data showed that after a twelve month period of being stored at 2-8°C the CPE formulation is just as effective as when it is new. The viability of cells remained similar over the twelve months the CPE formulation was stored. Cell death and proliferation rates remained within the same range.

Data were collected to compare the original CPE formulation with the new CPE 25/30 formulation. Studies were conducted at 0 and 3 months to test the effectiveness of the formulation over time as well as to test how well the new formulation works in relation to the old. The data showed that the CPE 25/30 formulation is as effective in inhibiting the growth of tumor cells as is the original CPE formulation. Cell viability was similar between HeLa cells treated with various concentrations of drug using either the CPE formulation or the CPE 25/30 formulation. Cell death and proliferation remained within the same range even over time.

Information was collected comparing the original CPE formulation with the CPE 33/27 formulation at time zero on HeLa cells. The data showed that CPE 33/27 had the same affects on HeLa cells in cell viability, percent of dead cells and proliferation rate.

**Table 6. Effect of M₄N in DMSO or the CPE Formulation on C-33A Cells**

| Treatment | % Viability | % Dead Cells | Proliferation Rate |
|---|---|---|---|
| None | 95.4 | 4.6 | 1.00 |
| 0 µM DMSO | 82.0 | 18.0 | 0.47 |
| 20 µM DMSO | 82.6 | 17.4 | 0.20 |
| 40 µM DMSO | 67.0 | 33.0 | 0.15 |
| 60 µM DMSO | 60.4 | 39.6 | 0.14 |
| 80 µM DMSO | 56.7 | 43.3 | 0.11 |
| 0 µM CPE | 92.8 | 7.2 | 0.79 |
| 20 µM CPE | 93.0 | 7.0 | 0.43 |
| 40 µM CPE | 89.1 | 10.9 | 0.43 |
| 60 µM CPE | 89.4 | 10.6 | 0.22 |
| 80 µM CPE | 77.5 | 22.5 | 0.15 |

**Table 7. Effect of M₄N in DMSO or the CPE Formulation on HeLa Cells at time 0**

| Treatment | % Viability | % Dead Cells | Proliferation Rate |
|---|---|---|---|
| None | 97.7 | 2.3 | 1.00 |
| 0 µM DMSO | 93.7 | 6.3 | 0.43 |
| 20 µM DMSO | 90.1 | 9.9 | 0.25 |
| 40 µM DMSO | 86.8 | 13.2 | 0.25 |
| 60 µM DMSO | 63.9 | 36.1 | 0.13 |
| 80 µM DMSO | 61.4 | 38.6 | 0.02 |
| 0 µM CPE | 96.3 | 3.7 | 1.03 |
| 20 µM CPE | 95.6 | 4.4 | 0.52 |
| 40 µM CPE | 90.6 | 9.4 | 0.28 |
| 60 µM CPE | 80.4 | 19.6 | 0.13 |
| 80 µM CPE | 78.5 | 21.5 | 0.13 |

**Table 8. Effect of M₄N in DMSO or the CPE formulation on HeLa Cells at 9 months**

| Treatment | % Viability | % Dead Cells | Proliferation Rate |
|---|---|---|---|
| None | 95.7 | 4.2 | 1.00 |
| 0 µM DMSO | 94.9 | 5.1 | 0.76 |
| 20 µM DMSO | 89.3 | 10.7 | 0.19 |
| 40 µM DMSO | 91.2 | 8.8 | 0.14 |
| 60 µM DMSO | 67.5 | 32.5 | 0.03 |
| 80 µM DMSO | 50.0 | 50.0 | 0.02 |
| 0 µM CPE | 95.6 | 4.4 | 0.72 |
| 20 µM CPE | 68.7 | 31.3 | 0.24 |
| 40 µM CPE | 72.8 | 27.2 | 0.10 |
| 60 µM CPE | 86.4 | 13.6 | 0.09 |
| 80 µM CPE | 88.1 | 11.9 | 0.08 |

**Table 9. Effect of M₄N in DMSO or the CPE formulation on HeLa Cells at 12 months**

| Treatment | % Viability | % Dead Cells | Proliferation Rate |
|---|---|---|---|
| None | 92.1 | 7.9 | 1.00 |
| 0 µM DMSO | 90.5 | 9.5 | 0.77 |
| 20 µM DMSO | 87.4 | 12.6 | 0.23 |
| 40 µM DMSO | 86.4 | 13.7 | 0.04 |
| 60 µM DMSO | 64.6 | 35.4 | 0.04 |
| 80 µM DMSO | 76.5 | 23.6 | 0.15 |
| 0 µM CPE | 95.6 | 4.4 | 1.50 |
| 20 µM CPE | 96.8 | 3.3 | 0.74 |
| 40 µM CPE | 95.0 | 5.0 | 0.21 |
| 60 µM CPE | 75.0 | 25.0 | 0.05 |
| 80 µM CPE | 52.8 | 47.2 | 0.03 |

**Table 10. Comparison of the CPE formulation and the CPE 25/30 Formulation in HeLa Cells**

| Treatment | % Viability | % Dead Cells | Proliferation Rate |
|---|---|---|---|
| None | 93.5 | 6.6 | 1.00 |
| 0µM CPE | 92.3 | 7.8 | 0.92 |
| 20µM CPE | 93.5 | 6.6 | 0.33 |
| 40µM CPE | 76.7 | 23.4 | 0.08 |
| 60µM CPE | 44.5 | 55.6 | 0.03 |
| 80µM CPE | 43.4 | 56.7 | 0.04 |
| 0µM CPE 25/30 | 90.7 | 9.3 | 0.81 |
| 20µM CPE 25/30 | 90.7 | 9.4 | 0.34 |
| 40µM CPE 25/30 | 89.1 | 11.0 | 0.31 |
| 60µM CPE 25/30 | 77.3 | 22.8 | 0.12 |
| 80µM CPE 25/30 | 54.8 | 45.2 | 0.07 |

**Table 11. Comparison of the CPE formulation and the CPE 25/30 Formulation in HeLa Cells at 3 months**

| Treatment | % Viability | % Dead Cells | Proliferation Rate |
|---|---|---|---|
| None | 95.8 | 4.2 | 1.00 |
| 0µM CPE | 95.0 | 5.0 | 0.91 |
| 20µM CPE | 91.0 | 9.0 | 0.39 |
| 40µM CPE | 83.5 | 16.5 | 0.09 |
| 60µM CPE | 56.9 | 43.1 | 0.03 |
| 80µM CPE | 71.0 | 29.0 | 0.03 |
| 0µM CPE 25/30 | 93.4 | 6.6 | 0.87 |
| 20µM CPE 25/30 | 88.1 | 11.9 | 0.39 |
| 40µM CPE 25/30 | 86.6 | 13.4 | 0.32 |
| 60µM CPE 25/30 | 76.9 | 23.1 | 0.11 |
| 80µM CPE 25/30 | 64.4 | 35.6 | 0.04 |

**Table 12. Comparison of CPE formulation and the CPE 33/27 formulation in HeLa cells at time zero**

| Treatment | % Viability | % Dead Cells | Proliferation Rate |
|---|---|---|---|
| None | 95.0 | 5.0 | 1.00 |
| 0µM CPE | 96.4 | 3.6 | 1.11 |
| 20µM CPE | 88.1 | 11.9 | 0.35 |
| 40µM CPE | 74.2 | 25.9 | 0.14 |
| 60µM CPE | 73.9 | 26.2 | 0.10 |
| 80µM CPE | 78.4 | 21.6 | 0.11 |
| 0µM CPE 33/27 | 95.4 | 4.7 | 0.92 |
| 20µM CPE 33/27 | 89.2 | 10.9 | 0.43 |
| 40µM CPE 33/27 | 86.4 | 13.6 | 0.19 |
| 60µM CPE 33/27 | 70.7 | 29.3 | 0.10 |
| 80µM CPE 33/27 | 75.0 | 25.0 | 0.09 |

### Example 3. Multiple lots of M₄N can be used and create the same results

Various lots of M₄N were tested to show the effectiveness of the drug of different lots. HeLa cells were treated with increasing amounts of M₄N: 0 µM, 20 uM, 40 µM, 60 µM and 80 µM, for 72 hr with the CPE formulation. Each formulation was added to total 1% of the growth media (Minimal Essential Medium with L-glutamine supplemented with 10% fetal bovine serum, 1mM sodium pyruvate, 1x non-essential amino acid solution, and 1,000 IU/mL penicillin/1,000 µg/mL streptomycin solution). Control cells were grown under the same conditions and were left untreated. After 72 hr of treatment or no treatment, the total number of cells and the number of live cells in each sample were counted, using the trypan blue exclusion method. The cell proliferation rate and the percentage of dead cells in each sample were analyzed. Results of this experiment are shown in Tables 13 and 14. These result show that regardless of the lot of M₄N used, the effectiveness of the drug remains the same.

**Table 13 Treatment of HeLa cells with various lots of M₄N (lot EM1001)**

| Treatment | % Viability | % Dead Cells | Proliferation Rate |
|---|---|---|---|
| None | 96.2 | 3.7 | 1.00 |
| 0µM CPE | 96.2 | 3.8 | 0.92 |
| 20µM CPE | 94.9 | 5.1 | 0.25 |
| 40µM CPE | 73.7 | 26.3 | 0.10 |
| 60µM CPE | 44.4 | 55.6 | 0.01 |
| 80µM CPE | 59.8 | 40.2 | 0.01 |

**Table 14. Treatment of HeLa cells with various lots of M₄N (lot EM1002)**

| Treatment | % Viability | % Dead Cells | Proliferation Rate |
|---|---|---|---|
| None | 95.9 | 4.1 | 1.00 |
| 0µM CPE | 96.1 | 3.8 | 0.65 |
| 20µM CPE | 88.0 | 12.0 | 0.32 |
| 40µM CPE | 74.2 | 25.8 | 0.11 |
| 60µM CPE | 41.9 | 58.1 | 0.05 |
| 80µM CPE | 40.3 | 59.7 | 0.03 |

### Example 4. Intravenous Infusion Toxicity Studies

The purpose of this study was to determine the maximum tolerable dose of tetra-O-methyl NDGA (M₄N) when administered by intravenous infusion to male and female Beagle dogs, supplied by Convince Research Products (Cumberland, Virginia, U.S.A). Two Beagle dogs, one male and one female, about 6 to 9 months old at first dose, were administered the cyclodextrin vehicle containing 20% (w/v) HP-β-CD and 50% PEG 300, on Study Day ("SD") 1, followed by escalating levels of M₄N prepared in the cyclodextrin vehicle (i.e., the test articles). The preformed cyclodextrin vehicle and test articles were stored at room temperature. Sterile water, used as diluent, was obtained from Baxter Health Care Corp. (Deerfield, Michigan, U.S.A) or Abbott Laboratories (North Chicago, Illinois, U.S.A.) and stored at room temperature.

The test article, cyclodextrin vehicle and diluent were considered 100% pure for formulation purposes. Dose formulations were prepared on each day of dosing by adding the appropriate amount of test article or cyclodextrin vehicle into a glass container using a sterile syringe. For each dose, an equal amount of sterile water was added to the M₄N formulation or cyclodextrin vehicle to form a 50:50 (v/v) dilution. No dilution was required for the 200 mg/kg dose. All formulations were mixed by gentle inversion to ensure that a proper solution was formed. Formulations were loaded into medication cassettes and stored at room temperature until needed.

M₄N was administered at 25 mg/kg on SD 3, 50 mg/kg on SD 5, and 100 mg/kg on SD 8. Only the female received the full 100 mg/kg dose; due to mechanical malfunctions caused by precipitation in the infusion line, the male dog received approximately 72 mg/kg (i.e., 72%) of the intended dose. Two additional Beagle dogs were administered a 200 mg/kg dose; however, mechanical difficulties ensued and the male dog received approximately 180 mg/kg (90%) of the intended dose, whereas the amount the female received could not be determined.

On SD13, dosing could not be completed due to a pump mechanical failure that may be related to the viscosity of the 12.5 mg/mL formulation. The unused formulation was transferred into amber glass vials and stored at room temperature until reformulated on SD15 by diluting 50:50 (v/v) with sterile water. The 6.25 mg/mL dilutions were then loaded into medication cassettes.

The animals used were acclimated to laboratory conditions for at least 7 days prior to the first dose and released from quarantine by a staff veterinarian. During that time, each animal was identified by the ear tattoo and a temporary number that was recorded on each cage label. Animals were cared for in the conventional manner and in accordance with the provisions of the USDA Animal Welfare Act, the PHS Policy on Humane Care and Use of Laboratory Animals and the U.S. Interagency Research Animal Committee Principles for the Utilization and Care of Research Animals.

Feed and water were provided *ad libitum,* unless otherwise noted. No contaminants were known to be present in the diet or water at levels that might have interfered with achieving the objectives of the study. Animals were provided with positive human interaction such as petting, scratching, and talking during dosing and when performing physical examinations. Due to the jugular vein catheterization, dogs were not exercised outside of their cages following surgery. Rubber Kong toys or nylon toys were provided inside the cages.

Using aseptic surgical techniques, the animals were catheterized with an in-dwelling catheter inserted into the jugular vein, and were treated prophylactic ally with an analgesic and antibiotic on the day of surgery and with antibiotics and/or analgesics for 8 days following surgery. The study summary is presented in Tables 15, 16 and 17.

**Table 15. Doses of M₄N Administered in PEG 300/HP-β-CD Formulation to Beagle Dogs in the Toxicity Study**

| Dose | Study Day | Dose Level (mg/kg) | M₄N concentration (mg/mL) | Infusion rate (mL/kg/hr) | Infusion Duration (hr) | Male Dog | Female Dog |
|---|---|---|---|---|---|---|---|
| 1 | 1 | 0 | 0 | 4 | 4 | Full dose | Full dose |
| 2 | 3 | 25 | 6.25 | 4 | 1 | Full dose | Full dose |
| 3 | 5 | 50 | 6.25 | 4 | 2 | Full dose | Full dose |
| 4 | 8 | 100 | 6.25 | 4 | 4 | 71.9% of dose | Full dose |
| 5a | 13 | 200 | 12.50 | 4 | 4 | none | none |
| 5b | 15 | 200 | 6.25 | 4 | 8 | 90.3% of dose | Unknown amount |

**Table 16. Individual Body Weights of Beagle Dogs (in Kg) Infused with M₄N**

| Group Sex | Animal No. | SD1 | SD3 | SD6 | SD8 | SD9 | SD13 | SD15 | SD17 |
|---|---|---|---|---|---|---|---|---|---|
| Male | 10529 | 7.9 | 7.7 | 7.8 | 7.7 | 7.7 | NA | NA | NA |
| Male | 10567 | NA | NA | NA | NA | NA | 10.2 | 10.0 | 10.0 |
| Female | 10530 | 8.3 | 8.3 | 8.3 | 8.2 | NA | NA | 8.1 | 8.2 |
| Female | 10568 | NA | NA | NA | NA | NA | 6.8 | 6.8 | 6.8 |

| | | | | | | | | | |
|---|---|---|---|---|---|---|---|---|---|
| SD = Study Day | | | | | | | | | |

**Table 17. Serum Concentrations of M₄N in Individual Beagle Dogs (in mg/mL) After Infusion of Various Doses (in mg/kg)**

| Animal No. | Sex | Study Day | M₄N Dose | Predose | Immediate | 0.5 hr post | 1 hr post | 2 hr post | 4 hr post | 8 hr post | 16 hr post |
|---|---|---|---|---|---|---|---|---|---|---|---|
| 10529 | Male | 3 | 25 | ND | 5.262 | 2.343 | 1.157 | 0.682 | 0.322 | 0.189 | 0.113 |
| 10529 | Male | 6 | 50 | 0.011 | 8.661 | 4.775 | 2.255 | 1.464 | 0.989 | 0.386 | 0.226 |
| 10529 | Male | 8 | 100 | 0.216 | | 7.416 | 3.728 | 2.034 | 1.68 | 0.322 | 0.357 |
| 10567 | Male | 13 | | <LLOQ | | | | | | | |
| 10567 | Male | 15 | 200 | 0.020 | 52.96 | 27.79 | 22.33 | 16.90 | 14.82 | 3.246 | 2.501 |
| 10530 | Female | 3 | 25 | <LLOQ | 13.91 | 4.941 | 2.918 | 1.632 | 1.542 | 0.492 | 0.179 |
| 10530 | Female | 6 | 50 | 0.108 | 16.36 | 6.828 | 6.623 | 3.513 | 2.901 | 0.741 | 0.407 |
| 10530 | Female | 8 | 100 | 0.157 | 14.50 | 9.848 | 9.726 | 4.494 | 1.793 | 1.456 | 0.589 |

| | | | | | | | | | | | |
|---|---|---|---|---|---|---|---|---|---|---|---|
| ND = none detected; <LLOQ = less than lower limit of quantitation | | | | | | | | | | | |

Doses 1, 2 and 3 were delivered without incident. On SD 8, during the 100 mg/kg dose, the syringe pumps stopped prematurely and only 88.6 mL of the intended 123.2 mL dose was delivered to the male dog (No. 10529). Pumps were manually restarted as needed, but only the female dog (No. 10530) received the entire dose.

On SD 15, a 200 mg/kg dose was attempted to be given to dogs No. 10567 and No. 10568 with a diluted test article formulation and an 8-hr target infusion duration. After 7 hr and 45 min. of dosing, the pump for the male dog stopped. The male dog (No. 10567) thus received only 289 mL of the intended 320 mL dose. After 2 hr and 12 min. of dosing, the infusion needle was found to be detached from the infusion line on the female dog (No. 10568) and dosing was discontinued. The amount of M4N delivered intravenously to the female dog could not be determined.

Following completion of each intravenous infusion, 1 mL of sterile saline was administered as a slow bolus injection to flush the test material from the infusion line into the animal, followed by an injection of heparin-saline solution to fill the infusion line to maintain patency. The animals were observed. Cageside observation included observation for mortality, morbundity, general health, and signs of toxicity. Clinical observations included evaluation of skin and fur characteristics, surgical site, eye and mucous membranes, respiratory, circulatory, autonomic and central nervous systems, and somatomotor and behavior patterns. Blood was collected and placed into 2.5 mL serum separator tubes for serum M₄N concentration analysis. The tubes were inverted several times, stored on wet ice, and centrifuged at about 3000 rpm for about 10 min. at 4°C. Serum was transferred to microcentrifuge tubes and stored at -75 ± 10° C.

Samples were shipped on dry ice to the Analytical Chemistry Laboratory of GeneLogic (Gaithersburg, Maryland, U.S.A.) for test article analysis. The method involved treating aliquots of dog serum, at least 0.5 mL, with acetonitrile, filtering, and injecting the filtrate onto an HPLC column with tandem mass-spectrometry detection (LC-MS/MS). The analyte was quantified using an external standard curve with triazolam as an internal standard.

Male dog No. 10529 and female dog No. 10530 were removed from study on SD 9 and 17, respectively, and euthanized and exsanguinated. Male No. 10567 and female No. 10568 were euthanized and exsanguinated on SD17. All animals were euthanized by an intravenous injection of Nembutal®.

All dogs were necropsied as soon as possible after the time of death. A gross necropsy, which included examination of the external surface of the body, surgical site, all orifices, the cranial, thoracic, and abdominal cavities and their contents, was performed. Bone marrow smears were prepared from femoral marrow. Slides were air-dried, fixed in methanol, and held for possible future evaluation. Tissues were preserved in 10% neutral buffered formalin ("NBF"). In addition, the infusion lines attached to the vascular access port ("VAP") were collected, knotted at both ends, and stored in NBF. The kidneys, lung, urinary bladder, and any gross lesions were embedded, sectioned, stained, and examined by a board certified veterinary pathologist. There were no test article related pathological fmdings in the examined tissues.

Results showed that treatment with M₄N had no effect on mortality. There were no apparent treatment-related clinical observations or effects on body weight. There were no test-article related gross pathology findings or microscopic changes. M₄N serum concentration levels were highest immediately following the infusion period and M₄N levels were higher in the female dog than in the male dog at all measured intervals. M₄N was still detectable 16 hr following the infusion period, with the amount detected increasing with increasing dose.

In conclusion, no treatment-related adverse effects were observed at doses of up to about 200 mg/kg.

### Example 5. Additional Intravenous Infusion Toxicity Studies

### A. A 7-Day Intravenous Infusion Toxicity Study of M₄N (CPE) in Beagle Dogs

This study was conducted to evaluate the toxicokinetic profile of M₄N formulated in 30% HP-β-CD and 25% PEG 300 when administered as a single intravenous infusion dose for seven consecutive days in beagle dogs. This study constitutes the first study of this particular formulation of M₄N in dogs and was executed to evaluate the toxicity and compare tolerability of a diluted formulation (10 mg/mL versus 5 mg/mL).

A total of twelve beagle dogs (8 male/4 female) were dosed with M₄N via a 54-minute (Group 2) or 108-minute (Groups 1, 3 and 4) intravenous infusion. All animals received seven doses, one per day over seven consecutive days.

The following parameters were evaluated: clinical observations and body weights. Test article analysis was performed on Study Days 1 and 7, blood samples (targeting a volume of 2.0 mL) were collected from each animal from an appropriate vessel at the following time points:

| Group | Study Day | Toxicokinetic Time Points |
|---|---|---|
| 1, 3 and 4 | 1 | Pre-dose, 1, 1.8 (end of infusion), 4, 6, 8 and 16 hours post-dose initiation |
| | 7 | Pre-dose, 1, 1.8 (end of infusion), 4, 6, 8, 16, 24 and 36 hours post-dose initiation |
| 2 | 1 | Pre-dose, 30 minutes and 0.9 (end of infusion), 2, 4, 8 and 16 hours post-dose initiation |
| | 7 | Pre-dose, 30 minutes and 0.9 (end of infusion), 2, 4, 8, 16, 24 and 36 hours post-dose initiation |

Gross necropsies were performed on Groups 1 and 3 animals and all injection sites were processed for histology. Detailed clinical observations revealed the following findings: transient reversible uncoordination of hindlimbs was observed in Groups 2, 3 and 4. The majority of these observations were observed in Groups 3 and 4. Discolored urine appeared in Groups 1 and 3. Since this finding was noted in the vehicle group, it may be vehicle-related. Three observations of swelling in the forelimb area were noted in Groups 1, 2 and 3 beginning on Day 3 and ending on Day 4. The finding is likely indicative of irritation due to multiple needle insertions in the same dosing area (cephalic vein). A single observation of rapid respiration was noted for the Group 3 female as an isolated incident and was not observed again. Finally, sporadic observations of diarrhea, emesis, mucoid feces and soft feces were noted. These observations are common in beagle dogs and were considered stress-induced and not test article-related.

The serum analysis revealed that the lowest peak test article concentration (nominally 18759 ng/mL) were in the Group 4 animals receiving 45 mg/kg (5 mg/mL). Higher peak concentrations (nominally 33420 ng/mL) were noted in the Group 2 animals receiving 45 mg/kg (10 mg/mL). As anticipated, the highest concentrations (nominally 46704 ng/mL) were noted in the Group 3 animals receiving 90 mg/kg (10 mg/mL). The time of maximum concentration for all groups was at the end of infusion (0.9 hours for Group 2 and 1.8 hours for Groups 3 and 4). No test article was observed in the samples of any Group 1 animals receiving vehicle. Concentration curves following one administration are presented in FIG. 3A (non-logarithmic scale) FIG. 3B (logarithmic scale).

Gross necropsies were performed on the Group 1 and 3 animals. Injection sites were collected and processed for histology. The histology revealed that the injection site of the controls appeared less affected than those of the treated animals and there was more thickening and discoloration at the site of injection in the treated animals when compared to controls. This suggests that the test article was more irritating than the vehicle. No other gross findings were observed in the animals necropsied.

### B. A 14-Day Repeat Intravenous Infusion Toxicity Study of M₄N (CPE) in Beagle Dogs with a 28-Day Recovery Period

Male and female Beagle dogs were administered CPE (12.5 mg/mL in 20% HP-β-CD in 50% PEG 300) via intravenous infusion for 14 days, with a 28-day recovery period to determine the reversibility of any treatment-related toxicity. Initially, 32 dogs (16/sex) were randomLy assigned to one of four groups (5/sex in Groups 1 and 4, 3/sex in Groups 2 and 3) and administered 20% HP-β-CD in 50% PEG 300 (placebo) or test article (CPE) at doses of 22.5, 45, or 90 mg/kg. All animals received a total of 14 daily doses by intravenous infusion. Following dosing, 3 dogs/sex/group were euthanized on SD 16 and the remaining 2 dogs/sex in Groups 1 and 4 were maintained through a 28-day recovery period.

Parameters evaluated during the study included mortality, clinical observations, body weight and body weight changes, food consumption, ophthalmology, cardiology, clinical pathology, organ weights, and macroscopic and microscopic pathology.

Treatment with CPE had no effect on mortality; all animals survived until scheduled termination. There were no effects on body weight, body weight change, or food consumption. No treatment-related ocular effects were noted. Transient evidence of reversible CNS activity (ataxia) was seen in one dog at 90 mg/kg on two occasions. All other clinical observations were considered isolated occurrences unrelated to treatment.

There were no test-article related effects on cardiology. At predose and on SD 13, one male treated with placebo had premature ventricular beats and on SD 1 one female treated with 45 mg/kg CPE had one atrial beat that was not conducted to the ventricles; none of these findings were considered treatment-related.

There were no apparent test-article related effects on clinical pathology parameters. Significantly lower absolute reticulocyte counts were noted in males treated with 22.5 mg/kg CPE on SD 15; however the change was minimal, similar to the predose value, and not considered biologically or toxicologically significant.

On SD 16, statistical significances in absolute spleen weights of males treated with 22.5 and 45 mg/kg CPE were related to individual animal variation in the control group (placebo). No significant differences were noted in relative organ weights.

Lesions noted at the infusion sites in all animals at necropsy on SD 16 were considered secondary to the intravenous infusion procedure and not treatment related. Red discoloration of the infusion site was noted in one female in the 45 mg/kg CPE group and correlated microscopically to multifocal, mild, intimal, vascular thickening, multifocal, mild vascular hyperplasia of the smooth myocyte, and a multifocal, mild, subacute, subcutaneous perivasculitis, indicating irritation caused by the dosing technique. Select male and female animals in the placebo and 90 mg/kg CPE groups exhibited diffuse, mild to moderate splenic congestion attributed to the mode of euthanasia. All other macroscopic and microscopic findings noted at the SD 16, 42, and 43 necropsies were considered incidental and not treatment-related.

In conclusion, no adverse treatment-related effects were noted following 14 days of intravenous infusion of CPE in Beagle dogs at does up to 90 mg/kg.

### C. IV Dose Ranging Study in Beagle Dogs

A single male dog was administered M₄N (12.5 mg/mL) in 20% HP-β-CD/50% PEG 300 via IV infusion (10 mL/kg/hour). Increased pressure in the infusion line and test formulation leakage from the syringe occurred. Dosing resulted in salivation, mydriasis, red-colored urine, severe ataxia, tremors, and convulsion. Because the observations were considered severe, the animal was considered moribund and scheduled for euthanization. Prior to euthanization, the dog appeared to be recovering; all previously noted clinical observations were markedly reduced in severity.

An identical dose was attempted in a single female dog at an infusion rate of 5 mL/kg/hour and resulted in uncoordinated/unbalanced movements and frequent urination following dosing. The dog recovered within 1 hour and 15 minutes of dosing and presented no continued or additional adverse clinical signs.

An additional male and female were dosed at 150 mg/kg and then at 125 mg/kg. Severe ataxia, nystagmus, red urine or feces, frequent urination, vocalization during dosing, dyspnea, emesis, and/or salivation occurred at these dose levels. Both animals recovered within 1 hour of dosing and no additional important clinical signs were subsequently noted.

Incidental clinical, cageside, post dose, or unscheduled observations seen at 200, 150, and 125 mg/kg included minimal to mild erythema of the nose and/or ears, excretory changes (soft and/or mucoid feces) and one instance each of clear nasal discharge and red vaginal discharge. These findings occurred infrequently and were considered common laboratory findings unrelated to treatment. One instance of paddling during dosing on SD 43 in the female receiving 125 mg/kg occurred and is attributed to the use of dosing slings and is also considered unrelated to treatment.

Two extra (naive) dogs were added to the study design and dosed at 75 mg/kg/day at an infusion rate of 5 mL/kg/hour, for a total of 12 doses in a 13-day interval. The only notable post dose observation was a single observation of red-brown urine in the female on SD 7; the significance of this event is unknown. Clinical observations included soft feces in the male and frothy white emesis in the female; these findings were infrequent or isolated incidences and are considered unrelated to treatment. A post dose observation of weakened limbs was noted in the male on SD 2 and is attributed to the use of sling restraint. Both dogs recovered from any noted clinical signs within 1 hour of dosing. Treatment with M₄N had no effect on body weights and no gross lesions were noted at necropsy. No other treatment-related observations were noted.

### D. An 8-Day IV Infusion Toxicity Study in Sprague-Dawley Rats

Escalating doses of 0, 100, 150, or 200 mg/kg M₄N in 10% HP-CD in PEG 300 (6.25 mg/mL) were administered by IV infusion once every four days to male and female Sprague-Dawley rats (3/each sex) at 8 mL/kg/hr. Following the initial four infusions the study was extended to include daily infusions of four hours duration for up to 14 days. No obvious mortality related to M₄N occurred. One male rat was found dead on the first day dosage during administration of the vehicle and one female rat was found dead on day 5 of the study at the end of the infusion of 100 mg/kg of the test material. These deaths were not considered related to the test article because for one rat no test article (only vehicle) was administered and for the other rat, this was a single event with other rats surviving multiple days of dosing at 200 mg/kg/day.

Clinical and necropsy observations that occurred were secondary to the ports and infusion lines. These observations included swelling in the neck or axilla and limited use of the hind limbs. During the continued daily dosing phase of the study, tremors did occur in one male and one female rat. Also, ptosis and hyperactivity occurred in one male and one female rat. These were the only clinical observations that may have been related to the 200 mg/kg/day dosage of the test article.

Both male and female rats gained weight throughout the study. Highest weight gains occurred following administration of the vehicle. Feed consumption values were similar following administration of the vehicle or test article.

Based on the limited number of rats evaluated in this study, administration of the vehicle or test article at doses as high as 200 mg/kg/day for up to eight consecutive days appeared to be tolerated by the rats, causing some adverse clinical observations following daily dosing. Most adverse clinical observations and all necropsy findings that did occur were considered related to the ports and tubing used to dose the rats. At sacrifice, all infusion lines had either moved outside of the jugular vein and/or were no longer patent and masses (assumed to be test article) were found where the ports/lines were located.

### Comparative data of example 6. Solubility of M₄N in Modified Celluloses

A 10 mL solution of 50% HP-P-CD (w/v) and 0.5% hydroxypropyl methylcellulose ("HPMC") (w/v) for use as a solubilizing agent and/or excipient was made as follows: 5.9 mL of water suitable for injection into animals ("WFI") were placed in a glass beaker containing a stir bar. The beaker was placed on a magnetic plate, and the stir bar was set to stir at medium speed. Five grams of BP-β-CD were added slowly to the stirring WFI, using a spatula to direct the HP-β-CD to the center of the beaker. The HP-β-CD solution was stirred for about 24 hr or until the HP-β-CD was dissolved completely upon visual inspection. The resulting solution measured about 9.4 mL. About 0.6 mL of WFI was added to this resulting solution to reach 10 mL, to produce a solution of 50% HP--CD (w/v). Fifty milligrams of HPMC were added to the 50% HP-β-CD solution and stirred for about 1 hr or until the HPMC was dissolved upon visual inspection. The final solution was stirred for about 1 hr and was then stored at room temperature, protected from light. This method of making modified cyclodextrins with modified celluloses may be scaled up or down to obtain the desired volume or concentration of HP-β-CD/HPMC solution. Other modified cyclodextrin/modified cellulose solutions may be similarly made, for example, by substituting HP-β-CD with other modified cyclodextrins, or HPMC with other modified celluloses, in the process described above.

A 10 mL solution of M₄N at a concentration of about 10 mg/mL in 50% HP-β-CD/0.5% HPMC was made as follows. About 10 mL of the 50% HP-β-CD/0.5% HPMC solution were added to a glass beaker containing a stir bar. The beaker was placed on a magnetic plate and the stir bar was set to stir at medium speed. About 100 mg of M₄N were slowly added to the 50% HP-β-CD/0.5% HPMC in the center of the beaker with the aid of a spatula. The M₄N/50% HP-β-CD/0.5% HPMC mixture was stirred for 24 hr or until all M₄N was uniformly suspended without any clumps being present. The M₄N/50% HP-β-CD/0.5% HPMC mixture was heated at about 90°C for about 30 min. (or longer if a larger volume of solution was desired, for example, 1 hr at 90°C for 500 mL of the M₄N/50% HP-β-CD/0.5% HPMC mixture), or longer as needed to ensure complete dissolution of M₄N. The M₄N/50% HP-β-CD/0.5% HPMC mixture was observed for presence of any undissolved M₄N by holding the beaker against a white background followed by a dark background, looking for presence of particulates. The final M₄N/50% HP-β-CD/0.5% HPMC solution was stored at room temperature and was kept protected from light. M₄N was dissolved in this 50% HP-β-CD/0.5% HPMC formulation at the 1 mg/mL and 10 mg/mL concentrations when heated at 90°C and remained in solution after cool down, with stability at room temperature for greater than 7 days. M₄N did not dissolve in this same formulation at the 50 mg/mL concentration even at 90°C.

The foregoing process may be scaled up or down to obtain the requisite volume or concentration of M₄N. Formulations containing M₄N in other cyclodextrin/cellulose solutions may be similarly made, such as, for example, by substituting HP-β-CD in the process described above with other cyclodextrins, or by substituting HPMC with other modified celluloses.

A 10 mL solution of 5% ethylcellulose ("EC") in ethanol (w/v) for use as a solubilizing agent and/or excipient was made as follows: 10 mL of 100% ethyl alcohol ("EtOH") were placed in a glass beaker containing a stir bar, covered by a round Teflon® cover. The beaker was placed on a magnetic plate, and the stir bar was set to stir at medium speed. Five hundred (500) milligrams of EC was added slowly to the stirring ethanol, using a spatula to direct the EC to the center of the beaker so as to prevent EC powder from sticking to the beaker wall. The EC solution was stirred for about 2 hr or until the EC was dissolved completely upon visual inspection. The final solution was stored at room temperature, and was protected from light.

This method of making modified cellulose solutions may be scaled up or down to obtain the desired volume or concentration. Other modified cellulose solutions may be similarly made, for example, by substituting EC with other modified celluloses, in the process described above.

A 10 mL solution of M₄N at a concentration of about 20 mg/mL in 5% EC (w/v) (the "EC formulation") was made as follows. About 10 mL of 5% EC formulation, made as described above, were added to a glass beaker containing a stir bar. The beaker was placed on a magnetic plate and the stir bar was set to stir at medium speed, and covered with a round Teflon® cover. About 200 mg of M₄N were slowly added to the 5% EC formulation in the center of the beaker with the aid of a spatula to prevent M₄N from sticking to the beaker wall. The M₄N/EC mixture was stirred for 2 hr or until all M₄N had dissolved or was uniformly suspended without any clumps being present. The M₄N/EC mixture was heated at about 60° C for about 30 min. (or longer if a larger volume of solution was desired, for example, 1 hr at 60° C for 500 mL of the M₄N/EC mixture), or longer as needed to ensure complete dissolution of M₄N. The M₄N/EC mixture or solution was observed for presence of any undissolved M₄N by holding the beaker against a white background followed by a dark background, looking for presence of particulates. The final M₄N/EC solution was stored at room temperature and was kept protected from light.

The foregoing process may be scaled up or down to obtain the requisite volume or concentration of M₄N and the heating temperature may be increased or decreased to achieve dissolution of M₄N. Formulations containing M₄N in other modified celluloses may be similarly made, such as, for example, HPMC, MC (methylcellulose), and CMC (carboxymethylcellulose). Results of the solubility of M₄N in the modified celluloses are set forth in Table 18.

Results showed that M₄N was soluble at 1 mg/mL in the EC formulation without application of heat, the solution being stable at room temperature for greater than 3 days. M₄N was soluble at the 10 mg/mL concentration at 40°C and remained in solution after cooling, this solution being stable at room temperature for greater than 3 days. M₄N was soluble in the EC formulation at the 20 mg/mL concentration at 60°C and remained in solution after cooling, this solution being stable at room temperature for greater than 3 days. M₄N at the 30 mg/mL concentration was soluble in the EC formulation at 60°C, but did not remain in solution upon cooling. Higher concentrations of M₄N, such as at the 50 mg/mL or 100 mg/mL levels, were soluble in the EC formulation at 90°C, but did not remain in solution upon cooling.

**Table 18. Solubility of M₄N in Formulations containing modified celluloses**

| Excipients | Excipient Concentrat-ion (in w/v unless otherwise stated) | Drug Concentration (in mg/mL unless otherwise stated) | Dissolution After Rotation | Dissolution After Heating | Dissolution After CoolDown | Stability Time at Room Temperature |
|---|---|---|---|---|---|---|
| | | | | | | |
| HPMC | 2.3% | 1 | N | N at 90°C | | |
| | | 10 | N | N at 90°C | | |
| | | 50 | N | N at 90°C | | |
| | | 100 | N | N at 90°C | | |
| | | | | | | |
| HP-β-CD and HPMC | 50% HP-β-CD, 0.5% HPMC | 1 | N | Y at 90°C | Y | > 7 days |
| | | 10 | N | Y at 90°C | Y | > 7 days |
| | | 50 | N | N at 90°C | | |
| | | | | | | |
| | 84% HP-β-CD, 1 % HPMC, Lyophilized | 150mg/g | powder | | | |
| | | | | | | |
| EC | 5% (w/v) in EtOH | 1 | Y | | | > 3 days |
| | | 10 | N | Y at 40°C | Y | > 3 days |
| | | 20 | N | Y at 60°C | Y | > 3 days |
| | | 30 | N | Y at 60°C | N | |
| | | 50 | N | Y at 90°C | N | |
| | | 100 | N | Y at 90°C | N | |
| | | | | | | |
| MC | 2% (w/v) | 1 | N | N at 90°C | | |
| (Low viscosity) | | 10 | N | N at 90°C | | |
| | | | | | | |
| CMC | 1% (w/v) | 1 | N | N at 90°C | | |
| (High Viscosity) | | 10 | N | N at 90°C | | |
| | | | | | | |
| CMC | 4% | 1 | N | N at 90°C | | |
| (Low Viscosity) | | 10 | N | N at 90°C | | |
| | | | | | | |

### Comparative data of example 7. SOLUBILITY OF M₄N IN WATER-INSOLUBLE LIPIDS OR WATER-SOLUBLE ORGANIC SOLVENTS

A 10 mL solution of M₄N at a concentration of about 50 mg/mL in sesame oil was made as follows. About 10 mL of sesame oil were added to a glass beaker containing a stir bar. The beaker was placed on a magnetic plate and the stir bar was set to stir at medium speed. About 500 mg of M₄N were slowly added to the sesame oil in the center of the beaker with the aid of a spatula to prevent M₄N from sticking to the beaker wall. The M₄N/sesame oil mixture was stirred for about 2 hr or until all M₄N had dissolved or was uniformly suspended without any clump being present. The M₄N/sesame oil mixture was heated at about 60°C for about 30 min. (or longer if a larger volume of solution was desired, for example, 1 hr at 60°C for 500 mL of the M₄N/sesame oil mixture), or longer as need to ensure complete dissolution of M₄N. The M₄N/sesame oil mixture or solution was observed for presence of any undissolved M₄N by holding the beaker against a white background followed by a dark background, looking for presence of particulates. If crystals formed, the M₄N /sesame oil solution could be heated again at 60°C for about 1 hr, with stirring, on a hot magnetic plate until all M₄N was dissolved back in solution. The final M₄N/sesame oil solution was stored at room temperature and was kept protected from light.

The foregoing process may be scaled up or down to obtain the requisite volume or concentration of M₄N and the heating temperature may be increased or decreased to achieve dissolution of M₄N. Formulations containing M₄N in other water-insoluble lipids may be similarly . made, such as, for example, by substituting sesame oil in the process described above with corn oil, olive oil, soybean oil, peppermint oil, or other solubilizing agents, and combinations thereof. Results are shown in Table 19.

Table 19 shows, for example, that M₄N was soluble in corn oil at 60°C at concentrations ranging from 1 mg/mL up to 100 mg/mL and, except at the 100 mg/mL level, remained in solution after cooling, the solutions being stable for greater than 3 days at the 1 and 10 mg/mL concentrations, for less than 3 days at the 20, 40 and 50 mg/mL levels and for less than 1 day at the 60 mg/mL level. Further, M₄N was soluble in olive oil at 60°C at the 30 mg/mL level but did not remain in solution after cooling.

In sesame oil, M₄N was soluble at room temperature at the 10 mg/mL level and at 60°C at 20 mg/mL, 30 mg/mL and 50 mg/mL concentrations, and remained in solution upon cooling. The 10 mg/mL and 20 mg/mL solutions were stable at room temperature for more than 3 days, the 30 mg/mL solution was stable at room temperature for less than 3 days, and the 50 mg/mL solution was stable at room temperature for less than 1 day.

A 10 mL solution of M₄N at a concentration of about 60 mg/mL in 85% sesame oil and 15% Tween® 20 was made as follows. About 8.5 mL of sesame oil were added to a glass beaker containing a stir bar. The beaker was placed on a magnetic plate and the stir bar was set to stir at medium speed. About 1.5 mL of Tween® 20 was slowly added to the center of the beaker. About 600 mg of M₄N were slowly added to the sesame oil/Tween® 20 mixture in the center of the beaker with the aid of a spatula to prevent M₄N from sticking to the beaker wall. The M₄N/sesame oil/Tween@ 20 mixture was stirred for about 2 hr or until all M₄N had dissolved or was uniformly suspended without any clumps being present. The M₄N/sesame oil/Tween® 20 mixture was heated at about 60°C for about 30 min. (or longer if a larger volume of solution was desired, for example, 1 hr at 60°C for 500 mL of the M₄N/sesame oil/Tween® 20 mixture), or longer as need to ensure complete dissolution of M₄N. The M₄N/sesame oil/Tween® 20 mixture was observed for presence of any undissolved M₄N by holding the beaker against a white background followed by a dark background, looking for presence of particulates. The final M₄N/sesame oil/Tween® 20 solution was stored at room temperature and was kept protected from light. If crystals were to form during storage, the M₄N /sesame oil/Tween® 20 solution could be heated again at 60°C, with stirring, on a hot magnetic plate until all M₄N dissolved back in solution.

This process may be scaled up or down to obtain the requisite volume or concentration of M₄N and the heating temperature may be increased or decreased to achieve dissolution of M₄N. Formulations containing M₄N in other water-insoluble lipids combined with non-ionic surfactants, ionic surfactants or water-soluble organic solvents may be similarly made, such as, for example, by substituting sesame oil or Tween® 20 in the process described above with corn oil, olive oil, soybean oil, peppermint oil, Tween® 80, d-alpha-tocopheryl polyethylene glycol 1000 succinate (TPGS), lecithin, PEG 300, PEG 400, PEG 400 monolaureate, glycerol, polyvinylpyrrolidone ("PVP"), propylene glycol ("PG"), or other solubilizing agents, and combinations thereof. Results of the solubility of M₄N in water-insoluble lipids are set forth in Table 19.

Table 19 shows, for example, that M₄N at 60 mg/mL was soluble in a formulation containing 85% sesame oil and 15% Tween® 20 at 55°C and 40 mg/mL of M₄N was soluble in the same formulation at 45°C, but M₄N did not stay in solution in these formulations upon cooling. In contrast, M₄N at 29 mg/mL was soluble in a slightly different formulation containing 89% sesame oil and 11% Tween® 20 at 60°C and remained in solution upon cooling, the solution being stable at room temperature for more than 7 days.

A 1 mL emulsion of M₄N at a concentration of about 2.9 mg/mL in saline was made as follows. About 0.9 mL of a 0.9% saline solution was placed in a 1.5 mL-sized polypropylene tube. About 0.1 mL of an M₄N solution at a concentration of 29 mg/mL in 89% sesame oil, 11 % Tween® 20 was added to the polypropylene tube. The M₄N/sesame oil/Tween® 20 solution or mixture in saline was vortexed vigorously for one minute. The emulsion was prepared by sonication of the M₄N/sesame oil/Tween® 20 solution for five minutes with a microtip probe adjusted to an amplitude of 60% maximum probe amplitude. The M₄N/sesame oil/Tween® 20 emulsion was observed for presence of any precipitated M₄N by holding the beaker against a white background followed by a dark background, looking for presence of particulates. The final M₄N/sesame oil/Tween® 20 emulsion was stored at room temperature and was kept protected from light.

This process may be scaled up or down to obtain the requisite volume or concentration of M₄N. Formulations containing M₄N in other lipids combined with other surfactants, or water-soluble organic solvents may be similarly made, such as, for example, by substituting sesame oil or Tween® 20 in the process described above with corn oil, olive oil, soybean oil, peppermint oil, Tween® 80, TPGS, lecithin, PG, PEG 300, PEG 400, PEG 400 monolaureate, glycerol, polyvinylpyrrolidone ("PVP"), or other solubilizing agents, and combinations thereof.

**Table 19. Solubility of M₄N in water-insoluble Lipids**

| Excipients | Excipient Concentration | Drug Concentration (in mg/mL unless otherwise stated) | Dissolution After Rotation | Dissolution After Heaving | Dissolution After CoolDown | Stability Time at Room Temperature |
|---|---|---|---|---|---|---|
| | | | | | | |
| Corn Oil | 100% | 1 | N | Y at 60°C | Y | > 3 days |
| | | 10 | N | Y at 60°C | Y | > 3 days |
| | | 20 | N | Y at 60°C | Y | <3 days |
| | | 40 | N | Y at 60°C | Y | <3 days |
| | | 50 | N | Y at 60°C | Y | < 3 days |
| | | 60 | N | Y at 60°C | Y | < 1 day |
| | | 100 | N | Y at 60°C | N | |
| | | | | | | |
| Olive Oil | 100% | 30 | N | Y at 60°C | N | |
| | | | | | | |
| Sesame Oil | 100% | 10 | Y | | | > 3 days |
| | | 20 | N | Y at 60°C | Y | > 3 days |
| | | 30 | N | Y at 60°C | Y | < 3 days |
| | | 50 | N | Y at 60°C | Y | < 1 day |
| | | | | | | |
| Peppermint Oil | | 1 | Y | | | > 3 days |
| | | 10 | Y | | | > 3 days |
| | | 20 | Y | | | > 3 days |
| | | 40 | N | Y at 40°C | Y | > 3 days |
| | | 60 | N | Y at 40°C | Y | < 3 days |
| | | 100 | N | Y at 40°C | Y | < 1 day |
| | | 125 | N | Y at 40°C | Y | < 1 day |
| | | | | | | |
| Soybean Oil | 100% | 10 | N | Y at 60°C | Y | > 7 days |
| | | 30 | N | Y at 60°C | N | |
| | | 50 | N | Y at 60°C | N | |
| | | | | | | |
| Mineral Oil | 100% | 10 | N | Y at 60°C | Y | > 7 days |
| | | 50 | N | Y at 60°C | Y | < 1 day |
| | | 100 | N | Y at 60°C | N | |
| | | 200 | N | Y at 60°C | N | |
| | | | | | | |
| Olive Oil, Soybean Oil | 80% Olive Oil, 20% Soybean Oil | 60 | N | Y at 70°C | N | |
| | | | | | | |
| Sesame Oil, Tween@ 20 and Glycerol | 75% Sesame Oil, 9% Tween® 20,16% Glycerol | 24.3 | N | Y at 60°C | Y | < 7 days |
| | 10% Oil emulsion in 90% saline | 2.4 | Y | | | <1 day |
| | | | | | | |
| Sesame Oil and Tween® 20 | 89% Sesame Oil,11% Tween® 20 | 29 | N | Y at 60°C | Y | > 7 days |
| | 10% Oil emulsion in 90% saline | 2.9 | Y | | | <3 days |
| | 85% Sesame Oil, 15% Tween® 20 | 40 | N | Y at 45°C | N | |
| | 85% Sesame Oil, 15% Tween® 20 | 60 | N | Y at 55°C | N | |
| | | | | | | |
| Peppermint Oil, PEG 300 | 50% Peppermint Oil, 50% PEG 300 | 40 | N | Y at 35°C | Y | > 7 days |
| | | 60 | N | Y at 35°C | Y | > 7 days |
| | | 125 | N | Y at 35°C | N | |
| | 60% Peppermint Oil, 40% PEG 300 | 60 | N | Y at 40°C | Y | > 3 days |
| | | | | | | |
| Peppermint Oil, PEG 400 | 50% Peppermint Oil, 50% PEG 400 | 40 | N | Y at 40°C | Y. | > 7 days |
| | | 60 | N | Y at 40°C | Y | > 7 days |
| | | 100 | N | Y at 40°C | N | |
| | | 125 | N | Y at 40°C | N | |
| | 60% Peppermint Oil, 40% PEG 400 | 60 | N | Y at 40°C | N | |
| | | | | | | |
| Peppermint Oil and Tween® 20 | 50% Peppermint Oil, 50% Tween® 20 | 40 | N | Y at 40°C | Y | > 3 days |
| | | 60 | N | Y at 40°C | Y | > 3 days |
| | | 125 | N | Y at 40°C | N | |
| | | | | | | |
| Peppermint Oil, PEG 400, Glycerol | 40% Peppermint Oil, 40% PEG 400, 20% Glycerol | 52 | N | Y at 40°C | N | |
| | 45% Peppermint Oil, 45% PEG 400, 10% Glycerol | 59 | N | Y at 40°C | N | |
| | | | | | | |
| Peppermint Oil and Sesame Oil | 50% Peppermint Oil, 50% Sesame Oil | 20 | Y | | | > 3 days |
| | | 40 | N | Y at 40°C | Y | > 3 days |
| | | 60 | N | Y at 40°C | Y | > 3 days |
| | | | | | | |
| Peppermint Oil, Tween® 20, PEG 400 | 33% Peppermint Oil, 33% Tween® 20, 33% PEG 400 | 60 | N | Y at 40°C | Y | > 3 days |
| | | | | | | |

Table 20 shows the results obtained for the solubility of M₄N in water-soluble organic solvents EtOH, PG, PEG 300, PEG 400, PEG 400 monolaureate, glycerol, PVP, and certain combinations thereof.

**Table 20. Solubility of M₄N in Water-Soluble Organic Solvents**

| Excipients | Excipient Concentration | Drug Concentration (in mg/mL) | Dissolution After Rotation | Dissolution After Heating | Dissolution After CoolDown | Stability Time at Room Temperature |
|---|---|---|---|---|---|---|
| | | | | | | |
| Ethanol | 100% | 1 | Y | | | > 3 days |
| | | 10 | N | N at 37°C | | |
| | | | | | | |
| PVP | 15% | 1 | N | N at 90°C | | |
| | | 10 | N | N at 90°C | | |
| | | | | | | |
| Propylene Glycol | 100% | 1 | N | Y at 55°C | Y | < 1 day |
| | | 10 | N | Y at 55°C | Y | < 1 day |
| | | 20 | N | Y at 55°C | Y | < 1 day |
| | | | | | | |
| PEG 400 | 100% | 25 | N | Y at 50°C | Y | < 7 days |
| | | 30 | N | Y at 50°C | Y | < 3 days |
| | | 40 | N | Y at 50°C | Y | < 1 day |
| | | 50 | N | Y at 60°C | Y | < 1 day |
| | | 100 | N | Y at 60°C | N | |
| | 5% | 10 | N | N at 50°C | | |
| | | | | | | |
| PEG 400 monolaurate | 100% | 20 | N | Y at 50°C | Y | > 3 days |
| | | 50 | N | Y at 50°C | Y | <1 day |
| | | | | | | |
| PEG 300 | 100% | 25 | N | Y at 50°C | Y | < 7 days |
| | | 30 | N | Y at 50°C | Y | < 3 days |
| | | 40 | N | Y at 50°C | Y | < 1 day |
| | | 50 | N | Y at 60°C | Y | < 1 day |
| | | 100 | N | Y at 60°C | N | |
| | 33% | 10 | N | N at 50°C | | |
| | | | | | | |
| Glycerol | 100% | 1 | N | Y at 70C | N | |
| | | 10 | N | Y at 70°C | N | |
| | | 20 | N | Y at 70°C | N | |
| | | | | | | |
| Propylene Glycol and Ethanol | 40%Propylene Glycol, 10% Ethanol | 10 | N | N | | |

### Comparative data of example 8. Solubility of _{M}4N in Non-Ionic Surfactants

A 10 mL solution of M₄N at a concentration of about 60 mg/mL in Tween® 20 was made as follows. About 10 mL of Tween® 20 were added to a glass beaker containing a stir bar. The beaker was placed on a magnetic plate and the stir bar was set to stir at medium speed. About 600 mg of M₄N were slowly added to the Tween® 20 in the center of the beaker with the aid of a spatula to prevent M₄N from sticking to the beaker wall. The MₜN/Tween® 20 mixture was stirred for 2 hr or until all M₄N had dissolved or was uniformly suspended without any clumps being present. The M₄N/Tween® 20 mixture was heated at about 60°C for about 30 inin. (or longer if a larger volume of solution was desired, for example, 1 hr at 60°C for 500 mL of the M₄N/Tween® 20 mixture), or longer as need to ensure complete dissolution of M₄N. The M₄N/Tween® 20 mixture or solution was observed for presence of any undissolved M₄N by holding the beaker against a white background followed by a dark background, looking for presence of particulates. The final M₄N/Tween® 20 solution was stored at room temperature and was kept protected from light. If crystals were to form during storage, the M₄N/Tween® 20 solution could be heated again at 60° C for about 1 hr, with stirring, on a hot magnetic plate or until all M₄N was dissolved back in solution.

This process may be scaled up or down to obtain the requisite volume or concentration of M₄N and the heating temperature may be increased or decreased to achieve dissolution of M₄N. Formulations containing M₄N in other non-ionic surfactant, ionic surfactants or amphiphilic molecules may be similarly made, such as, for example, by substituting Tween® 20 in the process described above with Tween® 80, other solubilizing agents, and combinations thereof. Results of the solubility of M₄N in Tween® 20, Tween® 80, and a combination of Tween® 20 and PEG 400 are shown in Table 21.

Table 21 shows that M₄N was soluble in Tween® 20 or Tween® 80 at a concentration of 1 mg/mL at room temperature. The M₄N in Tween® 20 solution ("M₄N/Tween® 20") was stable at room temperature for more than 7 days, while the M₄N in Tween® 80 solution ("M₄N/Tween® 80") was stable for more than 3 days of observation. Higher concentrations of M₄N were soluble in Tween® 20 or Tween® 80 at 50°C. Further, M₄N remained in solution after cooling at concentrations of up to 60 mg/mL in Tween® 20 or up to 50 mg/mL in Tween® 80, while becoming insoluble upon cooling at the 80 mg/mL and 100 mg/mL levels in Tween® 20. The 10 mg/mL and 20 mg/mL M₄N/Tween® 20 solutions were observed to be stable at room temperature for greater than 7 days. The 40 mg/mL and 80 mg/mL M₄N/Tween® 20 solutions were observed to be stable at room temperature for less than 3 days. For the M₄N/Tween® 80 solutions, the 10 mg/mL solution was observed to be stable at room temperature for more than 3 days while the 50 mg/mL solution was stable at room temperature for less than 1 day.

Results also show that M₄N was soluble in a combination of 50% Tween® 20 and 50% PEG 400, up to a concentration of 60 mg/mL of M₄N tested when heated at 65°C. M₄N remained in solution in these formulations upon cooling, the solutions being stable at room temperature for more than 3 days.

Table 22 shows the concentration of M₄N in µg/mL and its corresponding concentration in µM quantities.

**Table 21. Solubility of M₄N in Non-Ionic Surfactants**

| Excipients | Excipient concentration | Drug Concentration (in mg/mL) | Dissolution After Rotation | Dissolution After Heating | Dissolution After Cool Down | Stability at Room Temperature |
|---|---|---|---|---|---|---|
| | | | | | | |
| Tween® 20 | 100% (v/v) | 1 | Y | | | > 7 days |
| | | 10 | N | Y at 50°C | Y | > 7 days |
| | | 20 | N | Y at 50°C | Y | > 7 days |
| | | 40 | N | Y at 50°C | Y | < 3 days |
| | | 60 | N | Y at 50°C | Y | < 3 days |
| | | 80 | N | Y at 50°C | N | |
| | | 100 | N | Y at 50°C | N | |
| | | | | | | |
| Tween® 80 | 100% (v/v) | 1 | Y | | | > 3 days |
| | | 10 | N | Y at 50°C | Y | > 3 days |
| | | 50 | N | Y at 50°C | Y | < 1 day |
| | | | | | | |
| Tween® 20, PEG 400 | 50% Tween® 20, 50% PEG 400 | 30 | N | Y at 65°C | Y | > 3 days |
| | | 40 | N | Y at 65°C | Y | > 3 days |
| | | 50 | N | Y at 65°C | Y | > 3 days |
| | | 60 | N | Y at 65°C | Y | > 3 days |
| | | | | | | |

**Table 22. Conversion Table for concentration of M₄N**

| M₄N in µg/mL | M₄N in µM |
|---|---|
| 0 | 0 |
| 7.2 | 20 |
| 14.3 | 40 |
| 21.5 | 60 |
| 28.7 | 80 |

### Comparative data of example 9. Lyophilized Formulations Containing M₄N in HP-β-CD

A 120 mg lyophilized powder of M₄N at a concentration of about 185 mg/g (w/w) in HP-β=CD was made as follows. Equal molar amounts of HP-β-CD and M₄N were used to increase the complexation rate between HP-β-CD and M₄N. About 98 mg of HP-β-CD and about 22.2 mg M₄N were mixed together in a 1.5 mL-sized polypropylene tube. About 0.2 mL WFI was added to the mixture in the polypropylene tube containing the HP-β-CD/MaN powder mixture and vortexed for 1 minute to produce a HP-β-CD/M₄N suspension in water. The HP-β-CD/M₄N suspension was frozen at 20°C for 24 hours. The HP-β-CD/M₄N suspension was then centrifuged at 1,400 rpm under vacuum at 60°C for about 2 hours to remove all the water from the suspension. The dry powder of HP-β-CD/M₄N complex weighed about 120 mg. This HP-β-CD/M₄N powder complex may be then dissolved or resuspended in water or other solubilizing agents. The final M₄N/HP-β-CD powder complex was stored at room temperature and was kept protected from light. This process may be scaled up or down to obtain the requisite volume or concentration of M₄N. Formulations containing M₄N with other cyclodextrins may be similarly made, such as, for example, by substituting HP-β-CD in the process described above with other cyclodextrins. Results shown in Table 1 demonstrate that a powder complex of HP-β-CD/M₄N was obtained after lyophilization of the HP-β-CD/M₄N suspension consisting of about 81.5% HP-β-CD and 18.5% M₄N.

A 400 mg lyophilized powder of M₄N at a concentration of about 150 mg/g (w/w) in HP-β-CD/HPMC was made as follows. About 1 mL of a 50% HP-β-CD/0.5% HPMC solution, made as described above, was added to a 1.5 mL-sized polypropylene tube. About 60 mg M₄N were added to the polypropylene tube containing the HP-β-CD/HPMC suspension and vortexed for 1 minute to produce a HP-β-CD/HPMC/M₄N suspension. The HP-β-CD/HPMC/M₄N suspension was frozen at -20°C for 24 hours. The HP-β-CD/HPMC/M₄N suspension was then centrifuged at 1,400 rpm under vacuum at 60°C for about 5 hours to remove all the water from the suspension. The dry powder of HP-β-CD/HPMC/M₄N complex weighed about 400 mg. This HP-β-CD/HPMC/M₄N powder complex may be then dissolved or resuspended in water or other solubilizing agents. The final M₄N/HP-β-CD/HPMC powder complex was stored at room temperature and was kept protected from light. This process may be scaled up or down to obtain the requisite volume or concentration of M₄N. Formulations containing M₄N with other cyclodextrins/cellulose solutions may be similarly made, such as, for example, by substituting HP-β-CD in the process described above with other cyclodextrins, or by substituting HPMC with other modified celluloses. Results shown in Table 18 demonstrate that a powder complex of HP-β-CD/HPMC/M₄N was obtained after lyophilization of the HP-β-CD/HPMC/M₄N suspension consisting of about 84% HP-β-CD, 1% HPMC and 15% M₄N.

### Comparative data of example 10. Testing of IV Tubing for Delivery of M₄N

In this experiment, various types of tubing were tested for compatibility and for optimizing intravenous delivery of M₄N. The tests on compatibility were based on visual inspection and HPLC (high pressure liquid chromatography) analysis.

The visual inspection was conducted as follows: About 5 mL of a formulation to be tested ("formulated material") were passed through a tubing of about 20 cm in length. The formulated material was kept inside the tube for about 24 hr at room temperature (25°C). The formulated material was then circulated several times (about 5 times, for example) through the tubing and was collected in a beaker or a collection tube. The formulated material was carefully inspected for precipitates or particulates present, including by holding the beaker or collection tube against a white background followed by a dark background.

The compatibility test using HPLC analysis varied in the methodology depending on the tubing being tested, the length of time and the purpose of the tubing. This test measured the amount of M₄N in solution before and after circulation through different tubing.

The threshold for determining compatibility was 90% assay purity, meaning that if about 90% of M₄N remained in solution after interaction with the tubing and completion of the test, the material would be considered as being compatible. Any amount below 90% threshold was considered incompatible tubing. Results are summarized in Table 23.

This study shows that PTFE (polytetrafluoroethylene) and fluorelastomer (Barnant Company, Barrington, IL, U.S.A.), polypropylene (Cole-Parmer, Vernon Hills, IL, U.S.A.), FEP (fluorinated ethylene propylene) (Saint-Gobain, Mickleton, NJ, U.S.A.), PTFE (Cole-Parmer), polyethylene (Intramedic, Becton Dickinson, Sparks, MD, U.S.A.) and platinum cured silicone (small size) (Cole-Parmer) were all compatible for delivery of M₄N in the formulations herein described.

**Table 23. Compatible tubing materials for delivery of M₄N**

| COMPATIBLE TUBING | | | |
|---|---|---|---|
| | | | |

| TUBING TYPE | BRAND NAME | TUBING MANUFACTURER | CAT. NO. |
|---|---|---|---|
| PTFE and fluoroelastomer | CHEM-SURE | Barnant Company | 96210-16 |
| Polypropylene | | Cole-Parmer | 06500-02 |
| FEP | | Saint-Gobain | P288605 |
| PTFE | | Cole-Parmer | 06417-31 |
| Polyethylene | Intramedic | BD | 427420 |
| Platinum cured silicone (small size)** | | Cole-Parmer | 95802-01 |
| | | | |
| NONCOMPATIBLE TUBING | | | |
| | | | |

| TUBING TYPE | BRAND NAME | TUBING MANUFACTURER | CAT. NO. |
|---|---|---|---|
| Platinum cured silicone (manufacturing) | | Cole-Parmer | |
| Thermal set rubber Viton B (67% fluorine) | Viton | | |
| Polypropylene-based material with USP mineral oil | PharMed | | |
| Polyurethane | | Cole-Parmer | 96140-42 |
| Styrene-ethylene-butylene modified block copolymer with silicone oil | C-Flex | | |
| PVC (DEHP in pathway) | 180 | Nalgene | 8000-0002 |
| PVC (phthalate-free) | 580 | Nalgene | 8008-0004 |

| | | | |
|---|---|---|---|
| ** not preferred but somewhat compatible | | | |

### Example 11. Additional studies of Intravesicular Administration of Pharmaceutical Composition

One or more of the foregoing pharmaceutical compositions can be administered intravesicularly, such as for the treatment of carcinoma *in situ* of the urinary bladder, for example, at a dose of 800 mg every week for 6 weeks. The composition will contain an API, such as the NDGA compounds, e.g., M₄N. The API, such as M₄N, is present in the composition at 200 mg per 5 mL vial, and is stored in the refrigerator at 2°C - 8°C (36°F - 46°F). Prior to administration of the composition to a subject, the vial is removed from the refrigerator and is allowed to warm to room temperature without heating. The composition may be diluted by adding 800 mg (20 mL) to 55 mL of 0.9% Normal Saline Injection, USP. Bladder lavage is performed by intravesicular administration of the API (total volume of 75 mL when diluted as above), allowing the API to dwell for 2 hours, and then voided out. The appropriate compatible tubing is used, as described above.

Physicians or patients are advised that the composition containing the API is not to be given if bladder is injured, inflamed, or perforated, as systemic absorption will occur via loss of mucosal integrity. The compound is to be used with caution in patients with severe irritable bladder symptoms, as the API may cause symptoms of irritable bladder during instillation and dwell time. The patients are taught that the urine may be red-or pink-tinged for about 24 hr.

### Example 12. Additional studies of a human phase 0, three-way crossover microdose pharmacokinetic study of ¹⁴C-labelled M₄N in eight healthy male subjects

This study was designed to assess the absorption of M₄N when administered to humans as a sub-therapeutic dose either as a single oral dose under fed and fasted conditions or a single intravenous dose (Regimens A - C, respectively, in Table 24). The study was a three-way crossover study design in a target population of healthy male subjects and consisted of three study periods of approximately 35 hours duration, each separated by a minimum period of at least 7 days between dosing. During the course of each study period, pharmacokinetic blood samples were taken at specified time points after dosing and urine was collected over pre-defined time intervals. The subjects were able to leave the clinical unit after the completion of study specific procedures at 24 hours post-dose.

In this study, M₄N was administered to humans in a 100 µg quantity. M₄N was lightly-labelled with ¹⁴C (3.3 kBq per 100 µg) and administered to healthy volunteers. Each oral administration of M₄N consisted of 0.1 mg of ¹⁴C labeled M₄N and 376.8 mg of glycerol monooleate in a size 0 gelatin capsule. The single intravenous infusion of M₄N consisted of 0.1 mg/mL ¹⁴C labelled M₄N, 30% (w/v) HP-β-CD, and 25% (v/v) PEG diluted with water to 1mL for bolus injection. Following collection of blood and urine from each subject, samples were analyzed for ¹⁴C content using Accelerator Mass Spectrometry (AMS) to determine the maximum concentration of M₄N (Cₘₐₓ) that occurred at time Tₘₐₓ, the overall area under the curve (AUC) that corresponds to the overall absorption of M₄N at the times of testing (AUC₀₋ₜ) and overall (AUC_{0-∞}), the terminal half-life (T_{1/2}) of each sample and the relative and overall bioavailability (Fᵣₑₗ and F), of the oral dose of M₄N compared to the IV dose of M₄N. The mean ± SD values of pharmacokinetic parameters for ¹⁴C are presented in Table 24. The baseline levels of M₄N were corrected for any residual M₄N remaining in the subjects following the periods between dosing so as not to inflate the levels of M₄N for any subsequent dosing.

**Table 24. Mean ± SD values of pharmacokinetic parameters for ¹⁴C (Baseline Corrected)**

| Parameter | Regimen A (oral, fed) | Regimen B (oral, fasted) | Regimen C (Intravenous) |
|---|---|---|---|
| Cₘₐₓ (pmol/L) | 5.94 ± 1.33 | 9.29 ± 1.40 | 10.31 ± 1.77 |
| Tₘₐₓ (hours) | 2.50^{a} | 1.00^{a} | 0.08^{a} |
| AUC₀₋ₜ (pmol.h/L) | 88.0 ± 19.4 | 117.2 ± 9.2 | 96.6 ± 9.23 |
| AUC_{0-∞} (pmol.h/L) | 625.6 ± 183.7 | 416.6 ± 142.6 | 707.7 ± 380.4 |
| T½ (hours) | 96.5 ± 20.7 | 50.9 ± 22.4 | 116.2 ± 64.0 |
| Fᵣₑₗ (%) | 75.1 ± 16.2 | - | - |
| F (%) | 91.2 ± 19.1 | 122.1 ± 14.5 | - |

| | | | |
|---|---|---|---|
| ^{a}Median Regimen A: 100µg ¹⁴C-labelled M₄N (3.3 kBq) administered as a single oral dose following a high fat breakfast. Regimen B: 100µg ¹⁴C-labelled M₄N (3.3 kBq) administered as a single oral dose following an overnight fast. Regimen C: 100µg ¹⁴C-labelled M₄N (3.3 kBq) administered as 1 mL bolus intravenous solution following an overnight fast. | | | |

For the oral doses, the Cₘₐₓ values for total 14C were lower following oral administration of 100µg ¹⁴C-labelled M₄N after a high fat breakfast (Cₘₐₓ=5.94 ± 1.33pmol/L) than following oral administration after an overnight fast (Cₘₐₓ=9.29 ± 1.40pmol/L). Tₘₐₓ tended to occur later in fed subjects than in fasted subjects. In fed subjects, Tₘₐₓ, the time of occurrence of Cₘₐₓ, was highly variable and ranged from 1.5 to 24 hours post-dose, and in fasted subjects Tₘₐₓ generally occurred at 1 hour post-dose (range 0.50 to 2.00 hours). The AUC₀₋ₜ values were generally lower in fed subjects than in fasted subjects.

Following the intravenous dose the maximum concentration (Cₘₐₓ=10.31 ± 1.77pmol/L) occurred, as expected, at the first sampling time (0.08 hours post-dose) in eight of the ten subjects. In two subjects, Tₘₐₓ was 0.17 hours post-dose. The AUC₀₋ₜ values for total ¹⁴C plasma concentrations were slightly lower following the single oral dose in fed subjects than following the intravenous dose. Conversely, the corresponding AUC₀₋ₜ values were slightly higher following the single oral dose in the fasted subjects than following the intravenous dose.

The study formulations were well tolerated in both oral and intravenous administrations. There were no serious or severe adverse events and no subjects discontinued because of a study treatment related adverse event. No clinically significant changes in vital signs or ECGs were seen.

In conclusion regarding this study, the apparent absorption of M₄N was very high following oral administration in the fed and fasted state. In the presence of food, the rate and extent of absorption were lower compared with the fasted state and the time of occurrence of Cₘₐₓ was prolonged in the fed state. These conclusions are made on the assumption that the orally administered doses of ¹⁴C-labelled M₄N were not degraded prior to absorption.

### Example 13. Additional Studies of Solubility of M₄N in Water-soluble Organic Solvents

The solubility of M₄N in combinations of water-soluble organic solvents as noted in Table 25 was evaluated up to 48 hours. Following 2, 24 and 48 hours incubation at room temperature samples were analyzed via Reverse Phase-HPLC ("RP-HPLC") to quantify the solubility of M₄N. To prepare M₄N samples, 200 µL of 100 mg/mL M₄N dissolved in acetone were placed in 1.5 mL polypropylene microtubes. The solvent was allowed to evaporate at room temperature for 48 hours until the samples were completely dry.

The water miscible organic solvent formulations were prepared in 15 mL polypropylene centrifuge tubes. 10 mL of each formulation was prepared. Each solvent was added on the basis of weight using its respective density at 25°C. Following brief mixing, each formulation was filtered through a 0.45 µm surfactant free cellulose acetate ("SFCA") filter into a fresh 15 mL tube. Formulations were kept at room temperature until ready for use.

400 µL of each formulation combination (Table 25, where Benz = benzyl alcohol; Crem = Cremophor® EL; DMA = dimethylacetamide; T80 = Tween® 80) were added to the microtube, enabling a maximum solubility of 50 mg/Ml M₄N. The M₄N solubility was evaluated by RP-HPLC at 2, 24 and 48 hours incubation at room temperature. At each time point, the samples were centrifuged for 2 minutes at 13,000 rpm to pellet any solid M₄N. As noted in Table 25, over half of the formulation conditions examined were able to solubilize M₄N to a concentration of greater than 10 mg/Ml. The solubility of M₄N in glycerol at 2 and 48 hours was not detectable.

**Table 25. M₄N solubility in water miscible organic solvents up to 48 hours**

| | M₄N (mg/Ml) | | |
|---|---|---|---|
| | Time (hrs) | | |
| Composition | 2 | 24 | 48 |
| 100% EtOH | 7.20 | 7.22 | 7.91 |
| 100% PG | 1.10 | 1.33 | 1.76 |
| 100% PEG300 | 5.81 | 10.37 | 11.52 |
| 100% Glycerol | XXX | 0.08 | XXX |
| 100% Crem | 1.19 | 7.51 | 12.48 |
| 50% EtOH, 50% PG | 3.64 | 4.15 | 4.43 |
| 50% EtOH, 50% PEG300 | 10.94 | 15.07 | 16.61 |
| 50% EtOH, 50% Glycerol | 1.14 | 1.53 | 1.60 |
| 50% EtOH, 50% Crem | 13.95 | 17.58 | 18.49 |
| 50% EtOH, 50% T80 | 15.91 | 19.28 | 19.68 |
| 50% PG, 50% PEG300 | 2.65 | 4.88 | 5.30 |
| 50% PG, 50% Glycerol | 0.13 | 0.48 | 0.51 |
| 50% PG, 50% Crem | 2.94 | 6.33 | 7.20 |
| 50% PG, 50% T80 | 5.07 | 8.16 | 8.41 |
| 48%EtOH, 50%PG, 2%Benz | 4.42 | 4.79 | 4.92 |
| 48%EtOH, 50%PEG300, 2%Benz | 14.51 | 15.78 | 16.49 |
| 48%EtOH, 50%Glycerol, 2%Benz | 1.25 | 1.66 | 1.73 |
| 48%EtOH, 50%Crem, 2%Benz | 14.02 | 18.17 | 18.51 |
| 48%EtOH, 50%T80, 2%Benz | 16.17 | 19.55 | 19.96 |
| 44%EtOH, 50%PG, 6%DMA | 4.80 | 5.48 | 2.93 |
| 44%EtOH, 50%PEG300, 6%DMA | 15.12 | 18.61 | 18.27 |
| 44%EtOH, 50%Glycerol, 6%DMA | 1.43 | 1.90 | 2.01 |
| 44%EtOH, 50%Crem, 6%DMA | 14.85 | 20.42 | 21.08 |
| 44%EtOH, 50%T80, 6%DMA | 17.72 | 22.41 | 23.00 |
| 18%EtOH,30%PG,40%PEG300,10%T80,2%Benz | 7.84 | 9.79 | 10.16 |
| 18%EtOH,30%PG,40%Glyc,10%T80,2%Benz | 0.58 | 1.01 | 1.15 |
| 18%EtOH,30%PG,40%Crem,10%T80,2%Benz | 8.64 | 11.78 | 11.86 |
| 14%EtOH,30%PG,40%PEG300,10%T80,6%DMA | 9.40 | 10.55 | 11.30 |
| 14%EtOH,30%PG,40%Glyc,10%T80,6%DMA | 0.85 | 1.24 | 1.48 |
| 14%EtOH,30%PG,40%Crem,10%T80,6%DMA | 9.03 | 12.08 | 12.28 |
| 28%EtOH,30%PG,30%PEG300,10%T80,2%Benz | 8.87 | 10.19 | 10.27 |
| 28%EtOH,30%PG,30%Glyc,10%T80,2%Benz | 1.86 | 2.22 | 2.53 |
| 28%EtOH,30%PQ30%Crem,10%T80,2%Benz | 8.98 | 11.35 | 11.28 |
| 24%EtOH,30%PG,30%PEG300,10%T80,6%DMA | 9.91 | 10.80 | 10.67 |
| 24%EtOH,30%PG,30%Glyc,10%T80,6%DMA | 1.63 | 2.26 | 2.52 |
| 24%EtOH,30%PG,30%Crem,10%T80,6%DMA | 10.42 | 11.25 | 12.48 |

### Comparative data of example 14. M₄N Solubility in aqueous solutions

The solubility of M₄N in aqueous solutions containing either hydroxypropyl HP-β-CD or sulfobutyl ether β-cyclodextrin (SE-β-CD) (Captisol®, CyDex, Inc., Lenexa, KS, U.S.A.) was evaluated up to 48 hours at room temperature described above in Example 13. Ten 50 % solutions of HP-β-CD and SE-β-CD were prepared on a weight to volume basis. The M₄N for use in the samples was prepared as set forth in Example 13. Between 1.0 g and 5.0 g of either compound was weighed into a 10 mL volumetric flask on an OHAUS Analytical Plus Balance. Each sample was q.s. to 10 mL with water for injection (WFI). Following a 1 hour incubation at 40°C, the preparations were filtered through a 0.45 µm SFCA filter into a fresh 15 mL tube. Preparations were kept at room temperature until ready for use.

As shown in Table 26, the solubility of M₄N in WFI, 0.9% saline, 5% dextrose (D5W) was below the quantitation limit of the RP-HPLC method throughout the course of this study. Note the increased M₄N solubility as a function of HP-β-CD and Captisol® concentration and time.

**Table 26. M₄N solubility in aqueous solutions up to 48 hours**

| | M₄N (mg/mL) | | |
|---|---|---|---|
| | Time (hrs) | | |
| Composition | 2 | 24 | 48 |
| WFI | XXX | XXX | XXX |
| 0.9% Saline | XXX | XXX | XXX |
| D5W | XXX | XXX | XXX |
| 10% HP-β-CD | 0.35 | 0.45 | 0.46 |
| 20% HP-β-CD | 0.66 | 0.91 | 0.88 |
| 30% HP-β-CD | 1.38 | 1.97 | 2.02 |
| 40% HP-β-CD | 1.89 | 3.01 | 3.23 |
| 50% HP-β-CD | 2.52 | 4.95 | 4.98 |
| 10% Captisol® | 0.48 | 0.74 | 0.94 |
| 20% Captisol® | 0.76 | 1.45 | 1.39 |
| 30% Captisol® | 1.57 | 2.87 | 2.69 |
| 40% Captisol® | 1.42 | 3.90 | 4.15 |
| 50% Captisol® | 1.17 | 4.49 | 6.41 |

More than 20 formulation conditions using water miscible organic solvents were able to solubilize M₄N to a concentration of greater than 10 mg/mL. The solubility of M₄N in WFI, 0.9% saline, D5W was below the detection limit of the RP-HPLC method. The solubility of M₄N increases as a function of HP-β-CD and Captisol® concentration and time.

### Comparative data of example 15. M₄N Solubility in Hydroxypropyl β-cyclodextrin,

The aqueous solubility of M₄N at various concentrations of HP-β-CD was evaluated by the method reported by Higuchi and Connors (1965). Briefly, M₄N was accurately weighed and added in quantities exceeding its aqueous solubility were gently rotated (~12 rpm) at room temperature with aqueous solutions of HP-β-CD in increasing concentrations (0-350 mmol/L), for a period of 48 hours. The M₄N / HP-β-CD solutions were then filtered through a 0.45 µm SFCA filter and analyzed via RP-HPLC.

Although most drug/cyclodextrin complexes are thought to be inclusion complexes, cyclodextrins are also known to form non-inclusion complexes and complex aggregates capable of dissolving drugs through micelle-like structures. The phase-solubility profiles did not verify formation of inclusion complexes, but only detail how the increasing concentration of cyclodextrin influences drug solubility. Formation of the M₄N / HP-β-CD complex is non-linear, but accurate determination of stoichiometry (as well as stability constants) was not studied in the experiments of this Example, but could be determined by other means such as NMR or potentiometry.

### Example 16. M₄N Stability in HP-β-CD/PEG 300 buffer solutions

The stability of 10 mg/mL M₄N (prepared in a ratio of 75:25 40% HP-β-CD: 40 mg/mL M₄N PEG 300) in 15 mM buffer solutions was evaluated following incubation at 60°C.

Buffered 40 % solutions of HP-β-CD were prepared on a weight to volume basis. The M₄N for use in the samples was prepared as set forth in Example 13. 2.0 g HP-β-CD was weighed into 5 mL volumetric flasks on an OHAUS Analytical Plus Balance. 1 mL of a 100 mM buffer solution was added to each flask. Each sample was q.s. to 5 mL with WFI. Following a 1 hour incubation at 40°C, the preparations were filtered through a 0.45 µm SFCA filter into a fresh 15 mL tube. Preparations were kept at room temperature until ready for use.

750 µL 40% HP-β-CD buffered solution was placed in 1.5 mL polypropylene microtubes. 250 µL of a 40 mg/mL M₄N PEG 300 was added to each microtube enabling a solubility of 10 mg/mL M₄N. After gentle inversion of the sample tubes, the pH of each solution was measured using a Orion, Model 420A pH meter. An initial aliquot was removed for RP-HPLC analysis. Test samples were then placed in a Precision 60°C incubator. The M₄N stability was evaluated by RP-HPLC. At each time point, the samples were centrifuged for 2 minutes at 13,000 rpm to pellet any solid M₄N.

As shown in Table 27, a slight decrease in the concentration of the various M₄N solutions is observed following the 14 day incubation at 60°C. RP-HPLC data did not reveal an increase in sample impurities which could account for the magnitude of decrease in the concentration of M₄N. However, a pH-dependent change was observed in the M₄N impurities, yet these impurities made up less than 0.1% of the total peak area. Little if any changes are observed in the apparent sample pH during the incubation period (Table 27).

**Table 27. M₄N stability in HP-β-CD/PEG solutions up to 14 days at 60°C**

| Composition | M₄N (mg/mL) | | | | |
|---|---|---|---|---|---|
| | Time (days) | | | | |
| | 0 | 2 | 6 | 10 | 14 |
| WFI, 30% HP-β-CD, 25% PEG300 | 10.4 | 10.1 | 9.9 | 9.7 | 9.6 |
| 15 mM Phosphate, pH 3 30% HP-β-CD, 25% PEG300 | 10.3 | 10.1 | 9.6 | 9.7 | 9.6 |
| 15 mM Phosphate, pH 4 30% HP-β-CD, 25% PEG300 | 10.5 | 10.1 | 9.6 | 9.7 | 9.6 |
| 15 mM Acetate, pH 5 30% HP-β-CD, 25% PEG300 | 10.6 | 10.2 | 9.6 | 9.7 | 9.6 |
| 15 mM Acetate, pH 6 30% HP-β-CD, 25% PEG300 | 10.5 | 10.1 | 9.7 | 9.7 | 9.6 |
| 15 mM Phosphate, pH 7 30% HP-β-CD, 25% PEG300 | 10.5 | 10.3 | 9.7 | 9.7 | 9.7 |
| 15 mM Phosphate, pH 8 30% HP-β-CD, 25% PEG300 | 10.6 | 10.4 | 9.7 | 9.8 | 9.6 |
| 15 mM Phosphate, pH 9 30% HP-β-CD, 25% PEG300 | 10.4 | 10.2 | 9.7 | 9.8 | 9.7 |
| 15 mM Borate, pH 10 30% HP-β-CD, 25% PEG300 | 10.5 | 10.1 | 9.7 | 9.6 | 9.6 |
| 15 mM Borate, pH 11 30% HP-β-CD, 25% PEG300 | 10.5 | 10.3 | 9.8 | 9.5 | 9.4 |

A uniform decrease in stability regardless of apparent sample pH or buffer, as indicated by a loss in the recovery of M₄N, was observed after 14 days of incubation at 60°C.

### Example 17 - M₄N Stability in 11-14 mg/mL PEG300/HP-β-CD solutions

To support manufacturing specifications, this study examined the 24-hour room temperature stability of combinations of PEG300/HP-β-CD at varying M₄N target concentrations. Stock samples of M₄N stocks were prepared in 100% PEG 300 at drug concentrations of 33-56 mg/mL at 60°C as follows.

M₄N bulk drug was solubilized to concentrations of 33, 44, 48, 52, 55 and 56 mg/mL (w/w) in PEG 300 using the procedure set forth in Examples 13 and 16, following incubation of at least 2 hours at 60°C. Vigorous vortexing and mixing were necessary for complete solubilization of M₄N above 44 mg/mL. The M₄N stock solutions were filtered through a 0.45 µm SFCA filter and used within 30 minutes of preparation. Separately, a solution of 40% (w/v) HP-β-CD was prepared in sterile WFI and filtered. Combinations of the 40% HP-β-CD stock and the M₄N/PEG 300 stocks were combined in 1.5 mL polypropylene microtubes. The M₄N solubility was evaluated by RP-HPLC at 2 and 24 hours incubation at room temperature.

The requisite amount of M₄N stock was added to 40% HP-β-CD to yield the final concentrations of drug and excipients listed in Table 28. Samples were gently rotated (~12 rpm) at room temperature. At 2 and 24 hours of incubation, samples were centrifuged 2 minutes at 13,000 rpm and 50 µL aliquots were removed for RP-HPLC analysis. Regardless of target M₄N or formulation, little if any changes in solubility were observed after the 24 hour incubation (Table 28).

**Table 28 M₄N Stability in 11-14 mg/mL PEG300/HP-β-CD solutions**

| | Observed M₄N mg/mL | |
|---|---|---|
| Composition | t=2 hours | t=24 hours |
| 11 mg/mL M₄N, 25% PEG300,30% HP-β-CD | 11.3 | 11.3 |
| 12 mg/mL M₄N, 25% PEG300,30% HP-β-CD | 11.8 | 11.6 |
| 13 mg/mL M₄N, 25% PEG300,30% HP-β-CD | 12.7 | 12.7 |
| 14 mg/mL M₄N, 25% PEG300,30% HP-β-CD | 13.5 | 13.5 |
| 11 mg/mL M₄N, 33% PEG300,27% HP-β-CD | 11.4 | 11.3 |
| 11 mg/mL M₄N, 20% PEG300,32% HP-β-CD | 11.5 | 11.4 |

Test samples containing between 11-14 mg/mL M₄N formulated to a final concentration of 25% PEG 300 and 30% HP-β-CD were stable after 24 hours incubation at room temperature.

### Comparative data of example 18. 40 mg/mL M₄N/PEG300 Stability

The stability of 40 mg/mL M₄N dissolved in 100% PEG 300 was evaluated up to 24 hours incubation at 30°C, 45°C and 60°C. A stock 40 mg/mL M₄N in PEG 300 was prepared at 60°C, following the procedure set forth in Example 17. Subsequently, aliquots were removed and incubated at the appropriate temperature. Samples were rotated at 450 rpm during the entire course of incubation. Visual observations and RP-HPLC data were collected throughout. As shown in Table 29, after 6 hours incubation at 30°C tiny crystals were observed in the 40 mg/mL M₄N/PEG 300 formulation, with more appearing after 24 hours. The formation of crystals coincided with a loss of soluble M₄N (Table 30). The 40 mg/mL M₄N/PEG 300 samples incubated at 45°C and 60°C were stable after 24 hours incubation as assessed by visual observations and RP-HPLC analysis (Tables 29 and 30). No changes in the amount or types of impurities peaks were observed at any of the incubation temperatures.

**Table 29. Visual appearance of 40 mg/mL M₄N/PEG 300 stability samples**

| Incubation Condition | Visual Appearance | | | |
|---|---|---|---|---|
| | Time (hours) | | | |
| | 2 | 4 | 6 | 24 |
| 30°C | Clear | Clear | Few tiny crystals | Many tiny crystals |
| 45°C | Clear | Clear | Clear | Clear |
| 60°C | Clear | Clear | Clear | Clear |

**Table 30. 40 mg/mL M₄N/PEG 300 stability samples: RP-HPLC analysis**

| Incubation Condition | M₄N (mg/mL) | | | |
|---|---|---|---|---|
| | Time (hours) | | | |
| | 0 | 2 | 6 | 24 |
| 30°C | | 40.8 | 40.6 | 37.4 |
| 45°C | | 40.4 | 39.8 | 40.6 |
| 60°C | 39.5 | 39.1 | 39.2 | 39.2 |

After 6 hours incubation at 30°C tiny crystals were observed in.the 40 mg/mL M₄N/PEG 300 formulation. After 24 hours, even more crystals were observed as well as a >5% loss in soluble M₄N as determined by RP-HPLC analysis.

40 mg/mL M₄N/PEG 300 samples incubated at 45°C and 60°C were stable after 24 hours incubation as assessed by visual observations and RP-HPLC analysis.

## Claims

1. A composition for injection into animals comprising an active pharmaceutical ingredient and a pharmaceutically acceptable carrier, wherein the active pharmaceutical ingredient comprises tetra-O-methyl NDGA, and the carrier comprises a cyclodextrin and polyethylene glycol, the polyethylene glycol is present in the absence of ascorbic acid or butylated hydroxytoluene, and when the polyethylene glycol is polyethylene glycol 400, the polyethylene glycol 400 is present in the absence of polyethylene glycol 8000.

2. The composition of claim 1, wherein the composition is injectable intravenously into animals.

3. The composition of claim 1, wherein the composition comprises 0.1 mg to 200 mg of the active pharmaceutical ingredient.

4. The composition of claim 3, wherein the composition comprises 10 mg, 20 mg 25 mg, 30 mg, 40 mg, 50 mg, 60 mg, 75 mg, 100 mg or 200 mg of the active pharmaceutical agent.

5. The composition of claim 1, wherein the active pharmaceutical ingredient is present at a concentration of 1 mg/mL to 200 mg/mL.

6. The composition of claim 5, wherein the tetra-O-methyl NDGA is present at a concentration of 1 mg/mL, 2 mg/mL, 2.5 mg/mL, 5 mg/mL, 10 mg/mL, 12.5 mg/mL, 15 mg/mL, 20 mg/mL, 25 mg/mL, 30 mg/mL, 40 mg/mL, 50 mg/mL, 55 mg/mL, 60 mg/mL, 75 mg/mL, 100 mg/mL, 125 mg/mL, 150 mg/mL or 175 mg/mL.

7. The composition of claim 1, wherein the polyethylene glycol is PEG 300, PEG 400 or PEG monolaurate.

8. The composition of claim 1, wherein the polyethylene glycol is present at a concentration of 5% (v/v) to 100% (v/v).

9. The composition of claim 1, wherein the polyethylene glycol is PEG 300.

10. The composition of claim 9, wherein the PEG 300 is present at a concentration of 10% (v/v), 20% (v/v), 30% (v/v), 40% (v/v) or 50% (v/v).

11. The composition of claim 1, wherein the polyethylene glycol is PEG 400.

12. The composition of claim 11, wherein the PEG 400 is present at a concentration of 10% (v/v), 20% (v/v), 30% (v/v), 40% (v/v) or 50% (v/v).

13. The composition of claim 1 wherein the polyethylene glycol is PEG 400 monolaurate.

14. The composition of claim 13, wherein the PEG 400 monolaurate is present at a concentration of 20% (v/v) to 50% (v/v).

15. The composition of claim 1, wherein the carrier comprises an unmodified cyclodextrin or a modified cyclodextrin.

16. The composition of claim 15, wherein the modified cyclodextrin is selected from the group consisting of hydroxypropyl-β-cyclodextrin and sulfobutyl ether β-cyclodextrin.

17. The composition of claim 15, wherein the modified cyclodextrin is present at a concentration of 5% (w/v) to 80% (w/v).

18. The composition of claim 17, wherein the modified cyclodextrin is present at a concentration of 15% (w/v), 20% (w/v), 25% (w/v), 30% (w/v), 35% (w/v), 40% (w/v) or 50% (w/v).

19. The composition of claim 1, wherein the carrier comprises propylene glycol.

20. The composition of claim 1, wherein the carrier comprises glycerol.

21. The composition of claim 1, wherein the carrier comprises a surfactant selected from the group consisting of polysorbate, d-alpha-tocopheryl polyethylene glycol 1000 succinate and the reaction product of ethylene oxide and castor oil in a 35:1 molar ratio.

22. The composition of claim 21, wherein the surfactant is selected from the group consisting of polysorbate 20 and polysorbate 80.

23. The composition of claim 21, wherein the surfactant is present at a concentration of 5% (v/v) to 100% (v/v).

24. The composition of claim 1, wherein the carrier comprises a modified cellulose.

25. The composition of claim 24, wherein the modified cellulose is selected from the group consisting of ethyl cellulose, hydroxypropyl methylcellulose, methylcellulose and carboxy methylcellulose.

26. The composition of claim 24, wherein the modified cellulose is present at a concentration of 0.1% (w/v) to 10% (w/v).

27. The composition of claim 1, wherein the carrier comprises a water-insoluble lipid.

28. The composition of claim 27, wherein the water-insoluble lipid comprises a fat emulsion.

29. The composition of claim 28, wherein the fat emulsion is present at a concentration of 10% (w/v) to 30% (w/v).

30. The composition of claim 29, wherein the fat emulsion is present at a concentration of 20% (w/v).

31. The composition of claim 27, wherein the water-insoluble lipid is an oil.

32. The composition of claim 26, wherein the oil is at least one oil selected from the group consisting of corn oil, olive oil, peppermint oil, soybean oil, sesame seed oil, mineral oil and glycerol.

33. The composition of claim 27, wherein the water-insoluble lipid is an esterified fatty acid.

34. The composition of claim 1 for use as a medicament.

35. The composition of claim 1 for use in the treatment of a proliferative disease, hypertension, obesity, diabetes, a central nervous system disease, a neurodegenerative disease, Alzheimer's disease, dementia, amyotrophic lateral sclerosis, Parkinson's disease, stroke, an inflammatory disease, premalignant neoplasia, dysplasia or an infection.

36. The composition for use according to claim 35, wherein the composition is administered parenterally.

37. The composition for use according to claim 36, wherein the composition is administered by a route selected from the group consisting of intravenously, intra-arterially and intraperitoneally.

38. The composition for use according to claim 36, wherein the composition is administered intravenously.

39. The composition for use according to claim 35, wherein the proliferative disease is cancer or psoriasis.

40. The composition for use according to claim 35, wherein the inflammatory disease is selected from the group consisting of rheumatoid arthritis, osteoarthritis, ulcerative colitis, Crohn's disease, atherosclerosis, chronic obstructive pulmonary disease and multiple sclerosis.

41. The composition for use according to claim 35, wherein the disease is an intraepithelial neoplasia.

42. The composition for use according to claim 35, wherein the infection is a viral infection.

43. The composition for use according to claim 42, wherein the virus is selected from the group consisting of HIV, HTLV, HPV, HSV, HBV, EBV, Varicella-zoster virus, adenovirus, parvovirus and JC virus.

44. The composition for use according to claim 35, wherein the composition is administered at a dose of 10 mg of active pharmaceutical ingredient per kg weight of the subject to 600 mg of active pharmaceutical ingredient per kg weight of the subject.

45. The composition for use according to claim 35, wherein the composition is administered one or more times per week.

46. The composition for use according to claim 35, wherein the composition is administered one or more times per month.

47. A kit comprising the composition of claim 1 and instructions for use thereof.

## Patentansprüche

1. Zusammensetzung für die Injektion in Lebewesen, umfassend einen pharmazeutischen Wirkstoff und einen pharmazeutisch verträglichen Träger, wobei der pharmazeutische Wirkstoff Tetra-O-Methyl-NDGA umfasst, und der Träger ein Cyclodextrin und Polyethylenglykol umfasst, wobei das Polyethylenglykol in Abwesenheit von Ascorbinsäure oder butyliertem Hydroxytoluol vorhanden ist, und wobei das Polyethylenglykol Polyethylenglykol 400 ist, wobei Polyethylenglykol 400 in Abwesenheit von Polyethylenglykol 8000 vorhanden ist.

2. Zusammensetzung nach Anspruch 1, wobei die Zusammensetzung intravenös in Lebewesen injiziert werden kann.

3. Zusammensetzung nach Anspruch 1, wobei die Zusammensetzung 0,1 mg bis 200 mg des pharmazeutischen Wirkstoffs umfasst.

4. Zusammensetzung nach Anspruch 3, wobei die Zusammensetzung 10 mg, 20 mg, 25 mg, 30 mg, 40 mg, 50 mg, 60 mg, 75 mg, 100 mg oder 200 mg des pharmazeutischen Wirkstoffs umfasst.

5. Zusammensetzung nach Anspruch 1, wobei der pharmazeutische Wirkstoff in einer Konzentration von 1 mg/ml bis 200 mg/ml vorhanden ist.

6. Zusammensetzung nach Anspruch 5, wobei Tetra-O-Methyl-NDGA in einer Konzentration von 1 mg/ml, 2 mg/ml, 2,5 mg/ml, 5 mg/ml, 10 mg/ml, 12,5 mg/ml, 15 mg/ml, 20 mg/ml, 25 mg/ml, 30 mg/ml, 40 mg/ml, 50 mg/ml, 55 mg/ml, 60 mg/ml, 75 mg/ml, 100 mg/ml, 125 mg/ml, 150 mg/ml oder 175 mg/ml vorhanden ist.

7. Zusammensetzung nach Anspruch 1, wobei das Polyethylenglykol PEG 300, PEG 400 oder PEG-Monolaurat ist.

8. Zusammensetzung nach Anspruch 1, wobei das Polyethylenglykol in einer Konzentration von 5% (v/v) bis 100% (v/v) vorhanden ist.

9. Zusammensetzung nach Anspruch 1, wobei das Polyethylenglykol PEG 300 ist.

10. Zusammensetzung nach Anspruch 9, wobei PEG 300 in einer Konzentration von 10% (v/v), 20% (v/v), 30% (v/v), 40% (v/v) oder 50% (v/v) vorhanden ist.

11. Zusammensetzung nach Anspruch 1, wobei das Polyethylenglykol PEG 400 ist.

12. Zusammensetzung nach Anspruch 11, wobei PEG 400 in einer Konzentration von 10% (v/v), 20% (v/v), 30% (v/v), 40% (v/v) oder 50% (v/v) vorhanden ist.

13. Zusammensetzung nach Anspruch 1, wobei das Polyethylenglykol PEG 400-Monolaurat ist.

14. Zusammensetzung nach Anspruch 13, wobei das PEG 400-Monolaurat in einer Konzentration von 20% (v/v) bis 50% (v/v) vorhanden ist.

15. Zusammensetzung nach Anspruch 1, worin der Träger ein nicht modifiziertes Cyclodextrin oder ein modifiziertes Cyclodextrin umfasst.

16. Zusammensetzung nach Anspruch 15, wobei das modifizierte Cyclodextrin ausgewählt ist aus der Gruppe, bestehend aus Hydroxypropyl-β-Cyclodextrin und Sulfobutylether-β-Cyclodextrin.

17. Zusammensetzung nach Anspruch 15, wobei das modifizierte Cyclodextrin in einer Konzentration von 5% (w/v) bis 80% (w/v) vorhanden ist.

18. Zusammensetzung nach Anspruch 17, wobei das modifizierte Cyclodextrin in einer Konzentration von 15% (w/v), 20% (w/v), 25% (w/v), 30% (w/v), 35% (w/v), 40% (w/v) oder 50% (w/v) vorhanden ist.

19. Zusammensetzung nach Anspruch 1, worin der Träger Propylenglykol umfasst.

20. Zusammensetzung nach Anspruch 1, wobei der Träger Glycerin umfasst.

21. Zusammensetzung nach Anspruch 1, wobei der Träger ein Tensid umfasst, ausgewählt aus der Gruppe, bestehend aus Polysorbat, D-Alpha-Tocopheryl-Polyethylenglykol-1000-Succinat und dem Reaktionsprodukt von Ethylenoxid und Castoröl in einem Molverhältnis von 35:1.

22. Zusammensetzung nach Anspruch 21, wobei das Tensid ausgewählt ist aus der Gruppe, bestehend aus Polysorbat 20 und Polysorbat 80.

23. Zusammensetzung nach Anspruch 21, wobei das Tensid in einer Konzentration von 5% (v/v) bis 100% (v/v) vorhanden ist.

24. Zusammensetzung nach Anspruch 1, wobei der Träger eine modifizierte Cellulose umfasst.

25. Zusammensetzung nach Anspruch 24, wobei die modifizierte Cellulose ausgewählt ist aus der Gruppe, bestehend aus Ethylcellulose, Hydroxypropylmethylcellulose, Methylcellulose und Carboxymethylcellulose.

26. Zusammensetzung nach Anspruch 24, wobei die modifizierte Cellulose in einer Konzentration von 0,1% (w/v) bis 10% (w/v) vorhanden ist.

27. Zusammensetzung nach Anspruch 1, wobei der Träger ein wasserunlösliches Lipid umfasst.

28. Zusammensetzung nach Anspruch 27, wobei das wasserunlösliche Lipid eine Fettemulsion umfasst.

29. Zusammensetzung nach Anspruch 28, wobei die Fettemulsion in einer Konzentration von 10% (w/v) bis 30% (w/v) vorhanden ist.

30. Zusammensetzung nach Anspruch 29, wobei die Fettemulsion in einer Konzentration von 20% (w/v) vorhanden ist.

31. Zusammensetzung nach Anspruch 27, wobei das wasserunlösliche Lipid ein Öl ist.

32. Zusammensetzung nach Anspruch 26, wobei das Öl mindestens ein Öl ist, ausgewählt aus der Gruppe, bestehend aus Maisöl, Olivenöl, Pfefferminzöl, Sojabohnenöl, Sesamsamenöl, Mineralöl und Glycerin.

33. Zusammensetzung nach Anspruch 27, wobei das wasserunlösliche Lipid eine veresterte Fettsäure ist.

34. Zusammensetzung nach Anspruch 1 zur Verwendung als Medikament.

35. Zusammensetzung nach Anspruch 1 zur Verwendung bei der Behandlung einer proliferativen Erkrankung, von Bluthochdruck, Fettsucht, Diabetes, einer Krankheit des Zentralnervensystems, einer neurodegenerativen Krankheit, der Alzheimer-Krankheit, Demenz, von amyotropher Lateralsklerose, der Parkinson-Krankheit, von Schlaganfall, einer entzündlichen Erkrankung, von prämaligner Neoplasie, Dysplasie oder einer Infektion.

36. Zusammensetzung zur Verwendung nach Anspruch 35, wobei die Zusammensetzung parenteral verabreicht wird.

37. Zusammensetzung zur Verwendung nach Anspruch 36, wobei die Zusammensetzung über einen Weg verabreicht wird, der ausgewählt ist aus der Gruppe, bestehend aus intravenös, intraarteriell und intraperitoneal.

38. Zusammensetzung zur Verwendung nach Anspruch 36, wobei die Zusammensetzung intravenös verabreicht wird.

39. Zusammensetzung zur Verwendung nach Anspruch 35, wobei die proliferative Erkrankung Krebs oder Psoriasis ist.

40. Zusammensetzung zur Verwendung nach Anspruch 35, wobei die entzündliche Erkrankung ausgewählt ist aus der Gruppe, bestehend aus rheumatoider Arthritis, Osteoarthritis, Colitis ulcerosa, Morbus Crohn, Atherosklerose, chronischer obstruktiver Lungenerkrankung und Multipler Sklerose.

41. Zusammensetzung zur Verwendung nach Anspruch 35, wobei die Erkrankung eine intraepitheliale Neoplasie ist.

42. Zusammensetzung zur Verwendung nach Anspruch 35, wobei die Infektion eine virale Infektion ist.

43. Zusammensetzung zur Verwendung nach Anspruch 42, wobei das Virus ausgewählt ist aus der Gruppe, bestehend aus HIV, HTLV, HPV, HSV, HBV, EBV, Varizella-Zoster-Virus, Adenovirus, Parvovirus und JC-Virus.

44. Zusammensetzung zur Verwendung nach Anspruch 35, wobei die Zusammensetzung in einer Dosis von 10 mg des pharmazeutischen Wirkstoffs pro kg Gewicht des Subjekts bis 600 mg des pharmazeutischen Wirkstoffs pro kg Gewicht des Subjekts verabreicht wird.

45. Zusammensetzung zur Verwendung nach Anspruch 35, wobei die Zusammensetzung ein oder mehrere Male pro Woche verabreicht wird.

46. Zusammensetzung zur Verwendung nach Anspruch 35, wobei die Zusammensetzung ein oder mehrere Male pro Monat verabreicht wird.

47. Kit, umfassend die Zusammensetzung nach Anspruch 1 und Anweisungen zur Verwendung derselben.

## Revendications

1. Composition pour injection dans des animaux comprenant un ingrédient pharmaceutique actif et un véhicule pharmaceutiquement acceptable, dans laquelle l'ingrédient pharmaceutique actif comprend du tétra-O-méthyl-NDGA, et le véhicule comprend une cyclodextrine et du polyéthylèneglycol, le polyéthylèneglycol est présent en l'absence d'acide ascorbique ou d'hydroxytoluène butylé, et quand le polyéthylèneglycol est du polyéthylèneglycol 400, le polyéthylèneglycol 400 est présent en l'absence de polyéthylèneglycol 8000.

2. Composition de la revendication 1, laquelle composition est injectable par voie intraveineuse dans des animaux.

3. Composition de la revendication 1, laquelle composition comprend 0,1 mg à 200 mg de l'ingrédient pharmaceutique actif.

4. Composition de la revendication 3, laquelle composition comprend 10 mg, 20 mg, 25 mg, 30 mg, 40 mg, 50 mg, 60 mg, 75 mg, 100 mg ou 200 mg de l'ingrédient pharmaceutique actif.

5. Composition de la revendication 1, dans laquelle l'ingrédient pharmaceutique actif est présent à une concentration de 1 mg/mL à 200 mg/mL.

6. Composition de la revendication 5, dans laquelle le tétra-O-méthyl-NDGA est présent à une concentration de 1 mg/mL, 2 mg/mL, 2,5 mg/mL, 5 mg/mL, 10 mg/mL, 12,5 mg/mL, 15 mg/mL, 20 mg/mL, 25 mg/mL, 30 mg/mL, 40 mg/mL, 50 mg/mL, 55 mg/mL, 60 mg/mL, 75 mg/mL, 100 mg/mL, 125 mg/mL, 150 mg/mL ou 175 mg/mL.

7. Composition de la revendication 1, dans laquelle le polyéthylèneglycol est du PEG 300, du PEG 400 ou du monolaurate de PEG.

8. Composition de la revendication 1, dans laquelle le polyéthylèneglycol est présent à une concentration de 5 % (v/v) à 100 % (v/v).

9. Composition de la revendication 1, dans laquelle le polyéthylèneglycol est du PEG 300.

10. Composition de la revendication 9, dans laquelle le PEG 300 est présent à une concentration de 10 % (v/v), 20 % (v/v), 30 % (v/v), 40 % (v/v) ou 50 % (v/v).

11. Composition de la revendication 1, dans laquelle le polyéthylèneglycol est du PEG 400.

12. Composition de la revendication 11, dans laquelle le PEG 400 est présent à une concentration de 10 % (v/v), 20 % (v/v), 30 % (v/v), 40 % (v/v) ou 50 % (v/v).

13. Composition de la revendication 1, dans laquelle le polyéthylèneglycol est du monolaurate de PEG 400.

14. Composition de la revendication 13, dans laquelle le monolaurate de PEG 400 est présent à une concentration de 20 % (v/v) à 50 % (v/v).

15. Composition de la revendication 1, dans laquelle le véhicule comprend une cyclodextrine non modifiée ou une cyclodextrine modifiée.

16. Composition de la revendication 15, dans laquelle la cyclodextrine modifiée est sélectionnée dans le groupe constitué par l'hydroxypropyl-β-cyclodextrine et la sulfobutyléther-β-cyclodextrine.

17. Composition de la revendication 15, dans laquelle la cyclodextrine modifiée est présente à une concentration de 5 % (p/v) à 80 % (p/v).

18. Composition de la revendication 17, dans laquelle la cyclodextrine modifiée est présente à une concentration de 15 % (p/v), 20 % (p/v), 25 % (p/v), 30 % (p/v), 35 % (p/v), 40 % (p/v) ou 50 % (p/v).

19. Composition de la revendication 1, dans laquelle le véhicule comprend du propylèneglycol.

20. Composition de la revendication 1, dans laquelle le véhicule comprend du glycérol.

21. Composition de la revendication 1, dans laquelle le véhicule comprend un tensioactif sélectionné dans le groupe constitué par du polysorbate, du succinate de d-alpha-tocophérylpolyéthylèneglycol 1000 et le produit de la réaction entre de l'oxyde d'éthylène et de l'huile de ricin dans un rapport molaire 35:1.

22. Composition de la revendication 21, dans laquelle le tensioactif est sélectionné dans le groupe constitué par le polysorbate 20 et le polysorbate 80.

23. Composition de la revendication 21, dans laquelle le tensioactif est présent à une concentration de 5 % (v/v) à 100 % (v/v).

24. Composition de la revendication 1, dans laquelle le véhicule comprend une cellulose modifiée.

25. Composition de la revendication 24, dans laquelle la cellulose modifiée est sélectionnée dans le groupe constitué par l'éthylcellulose, l'hydroxypropylméthylcellulose, la méthylcellulose et la carboxyméthylcellulose.

26. Composition de la revendication 24, dans laquelle la cellulose modifiée est présente à une concentration de 0,1 % (p/v) à 10 % (p/v).

27. Composition de la revendication 1, dans laquelle le véhicule comprend un lipide insoluble dans l'eau.

28. Composition de la revendication 27, dans laquelle le lipide insoluble dans l'eau comprend une émulsion de graisse.

29. Composition de la revendication 28, dans laquelle l'émulsion de graisse est présente à une concentration de 10 % (p/v) à 30 % (p/v).

30. Composition de la revendication 29, dans laquelle l'émulsion de graisse est présente à une concentration de 20 % (p/v).

31. Composition de la revendication 27, dans laquelle le lipide insoluble dans l'eau est une huile.

32. Composition de la revendication 26, dans laquelle l'huile est au moins une huile sélectionnée dans le groupe constitué par l'huile de maïs, l'huile d'olive, l'huile essentielle de menthe, l'huile de soja, l'huile de graines de sésame, l'huile minérale et le glycérol.

33. Composition de la revendication 27, dans laquelle le lipide insoluble dans l'eau est un acide gras estérifié.

34. Composition de la revendication 1 pour utilisation comme médicament.

35. Composition de la revendication 1 pour utilisation dans le traitement d'une maladie proliférative, de l'hypertension, de l'obésité, du diabète, d'une maladie du système nerveux central, d'une maladie neurodégénérative, de la maladie d'Alzheimer, de la démence, de la sclérose latérale amyotrophique, de la maladie de Parkinson, de l'accident vasculaire cérébral, d'une maladie inflammatoire, d'une néoplasie prémaligne, d'une dysplasie ou d'une infection.

36. Composition pour utilisation selon la revendication 35, laquelle composition est administrée par voie parentérale.

37. Composition pour utilisation selon la revendication 36, laquelle composition est administrée par une voie sélectionnée dans le groupe constitué par la voie intraveineuse, la voie intraartérielle et la voie intrapéritonéale.

38. Composition pour utilisation selon la revendication 36, laquelle composition est administrée par voie intraveineuse.

39. Composition pour utilisation selon la revendication 35, la maladie proliférative étant le cancer ou le psoriasis.

40. Composition pour utilisation selon la revendication 35, la maladie inflammatoire étant sélectionnée dans le groupe constitué par la polyarthrite rhumatoïde, l'arthrose, la rectocolite hémorragique, la maladie de Crohn, l'athérosclérose, la bronchopneumopathie chronique obstructive et la sclérose en plaques.

41. Composition pour utilisation selon la revendication 35, la maladie étant la néoplasie intraépithéliale.

42. Composition pour utilisation selon la revendication 35, l'infection étant une infection virale.

43. Composition pour utilisation selon la revendication 42, le virus étant sélectionné dans le groupe constitué par le VIH, le HTLV, le VPH, le HSV, le VHB, l'EBV, le virus varicelle-zona, l'adénovirus, le parvovirus et le virus JC.

44. Composition pour utilisation selon la revendication 35, laquelle composition est administrée à une dose de 10 mg d'ingrédient pharmaceutique actif par kg de poids du sujet à 600 mg d'ingrédient pharmaceutique actif par kg de poids du sujet.

45. Composition pour utilisation selon la revendication 35, laquelle composition est administrée une ou plusieurs fois par semaine.

46. Composition pour utilisation selon la revendication 35, laquelle composition est administrée une ou plusieurs fois par mois.

47. Trousse comprenant la composition de la revendication 1 et des instructions pour l'utiliser.
